# EUROPEAN PATENT SPECIFICATION

(11) **EP 3 183 224 B1**
(45) Date of publication and mention of the grant of the patent: **25.02.2026**
(21) Application number: 15762666.4
(22) Date of filing: 24.08.2015
(51) Int. Cl.: C03C 11/00, C03C 12/00, C03B 19/10, C04B 35/447, C04B 38/00, A61K 9/16, A61L 27/10, A61L 27/12

(54) **POROUS BODIES**
PORÖSE KÖRPER
CORPS POREUX

(30) Priority: 22.08.2014 GB 201415005
(43) Date of publication of application: 28.06.2017
(73) Proprietor: The University Of Nottingham, Nottingham, Nottinghamshire NG7 2RD (GB)
(72) Inventor: AHMED, Ifty, Nottingham Nottinghamshire NG7 2RD (GB)
(74) Representative: Barker Brettell LLP
(86) International application number: PCT/GB2015/052446
(87) International publication number: WO 2016/027110

(56) References cited:
- WO-A1-2014/168631
- US-A- 4 661 137
- US-A- 5 011 677
- US-A1- 2001 024 662
- US-A1- 2006 122 049
- US-A1- 2006 274 989
- US-A1- 2010 064 726
- US-A1- 2013 344 337
- US-A1- 2014 212 626
- US-B1- 6 379 648
- US-B2- 6 358 532

## Description

The invention relates to powders comprising porous and/or non-porous bodies, in particular porous or non-porous spherical bodies or microspheres. The invention also relates to methods of manufacture of and the use of powders comprising porous and/or non-porous bodies such as resorbable porous microspheres.

Porous bodies can be useful in a range of applications including in the delivery of biological cells, growth factors, proteins and pharmaceutically active agents. By making the porous bodies from a resorbable material with controlled degradation, the resorbable bodies may be resorbed by their *in situ* environment over time. For instance, a bio-resorbable material may be suitable for being resorbed within a human or animal body. Consequently, a temporary medical device made from a bio-resorbable material may be left in the body to be resorbed over time. During resorption of the temporary medical device, specific and/or therapeutic agents, e.g. ions, may be released in a controlled manner.

The potential uses for resorbable and non-resorbable microspheres are many and varied. However, a reliable and reproducible method for manufacturing significant volumes of suitable porous microspheres has not yet been developed. Previous methods of manufacturing porous microspheres have generally been unsatisfactory. Typically, yields have been poor in terms of sphericity and/or porosity. The methods may also have relatively poor reliability and/or may produce microspheres lacking in uniformity. The methods may also be time consuming and/or may comprise several steps pre- and/or post-microsphere production.

US2014/0212626A1 discloses a process for making inorganic, metal oxide spheres that includes exposing solidified, molded microparticles that include a glass precursor composition to a temperature sufficient to transform the molded microparticles into molten glass and cooling the molten glass to form inorganic, metal oxide spheres.

US2010/0064726A1 discloses a process and apparatus for enhancing the formation of a uniform population of hollow glass microspheres. A burner head is used which directs incoming glass particles away from the cooler perimeter of the flame cone of the gas burner and distributes the glass particles in a uniform manner throughout the more evenly heated portions of the flame zone. As a result, as the glass particles are softened and expand by a released nucleating gas so as to form a hollow glass microsphere, the resulting hollow glass microspheres have a more uniform size and property distribution as a result of experiencing a more homogenous heat treatment process.

US2013/344337A1 discloses hollow microspheres comprising: at least 45 wt % of recycled glass based on the total weight of a feed composition from which the hollow microspheres are derived, wherein the hollow microspheres have a density of less than 1.25 g/cm³, strength at 20% volume reduction greater than 20 MPa and have a substantially single cell structure. US2013/344337A1 also provides hollow microspheres comprising: a blend of recycled glass and glass feed, wherein the hollow microspheres have a density of less than 1.25 g/cm³ and are made from a feed composition that is essentially free of an added effective blowing agent. US2013/344337A1 also provides a method for making hollow microspheres.

A first aspect of the invention provides a method of manufacture of a powder comprising, or consisting essentially of, porous microspheres, the method comprising:
providing a feed powder;
mixing the feed powder with one or more blowing agents to provide a mixture comprising feed powder particles and particles of one or more blowing agents; and
applying at least one spheroidisation flame to the mixture, so as to produce the powder.

The feed powder may comprise porous and/or non-porous particles. The feed powder particles may be resorbable or non-resorbable. The feed powder may comprise substantially spherical and/or non-spherical particles. The feed powder may have an average particle size of from 30 µm to 500 µm. In an embodiment, the feed powder may have an average particle size of at least 50 µm and/or an average particle size of up to 400 µm or up to 350 µm.

In an embodiment, the microspheres may comprise a glass, a ceramic or a glass-ceramic composition.

The microspheres may comprise a resorbable, e.g. a bio-resorbable composition, or a non-resorbable composition. The microspheres may comprise an at least partially resorbable composition or a fully resorbable composition. The microspheres may comprise a biocompatible composition.

The method of manufacture may comprise flame-spraying spheroidisation. In flame-spraying spheroidisation, the mixture may be sprayed into and/or through the spheroidisation flame. Typically, flame-spraying spheroidisation may produce a good yield of relatively uniform, highly spherical microspheres.

The sphericity and the porosity of the porous microspheres manufactured in accordance with the invention may depend on a number of factors, including the size and temperature of the spheroidisation flame and the residence time of the mixture within the spheroidisation flame. Accordingly, the size and/or temperature of the flame and/or the residence time may be controlled and/or varied in order to manufacture porous microspheres having desired properties.

The spheroidisation flame may be applied to the mixture for a predetermined period of time.

The spheroidisation flame temperature may be from 1900 °C to 3400 °C, depending on the type and ratio of fuel used.

An oxygen:butane spheroidisation flame may have a temperature of around 1920 °C. An oxygen:propane spheroidisation flame may have a temperature of around 2800 °C. An oxygen:acetylene spheroidisation flame may have a temperature of around 3400 °C.

The spheroidisation flame may be produced by an acetylene torch or a flame spray gun such as a plasma spray gun.

In an embodiment, the spheroidisation flame may be produced by an acetylene torch using an oxygen to acetylene ratio of 4:3.

The or each blowing agent may have an average particle size of at least 5 µm and/or up to 500 µm. By varying the particle size of the blowing agent(s), the size of the pores in the microspheres may be controlled. Different pore sizes may be achieved by using differently sized blowing agent particles. The size of the blowing agent particles may be varied.

The or each blowing agent may comprise a carbonate or a sulphate. For instance, suitable blowing agents may include calcium carbonate, strontium carbonate, zinc carbonate, magnesium carbonate, sodium sulphate and/or calcium sulphate. The type and/or amount of blowing agent(s) utilised can be used to control the levels of porosity and pore sizes of the porous microspheres manufactured in accordance with the invention. Accordingly, different types and/or amounts of blowing agent(s) may be selected in order to manufacture porous microspheres having desired properties.

In an embodiment, the ratio by weight of the blowing agent(s) to the feed powder particles may be from 5:1 to 1:10.

The mixture may be produced prior to applying the spheroidisation flame or at the same time as applying the spheroidisation flame. For instance, the mixture may have been formed before being supplied to a spray head configured to spray the mixture through the spheroidisation flame. Alternatively, the mixture could be formed at the spray head, e.g. by supplying the feed powder and the blowing agent(s) separately to the spray head. Alternatively, the mixture could be formed during spraying, e.g. by spraying the feed powder through a first spray head and the blowing agent(s) through one or more further spray heads such that the feed powder and the blowing agent(s) can mix together.

The method may comprise the step of coating the feed powder with the blowing agent(s).

The method may comprise the step of soaking the feed powder in a solution containing the blowing agent(s).

The solution containing the blowing agent(s) may be an aqueous solution.

The feed powder may be soaked in the solution containing the blowing agent(s) for a period of at least a few minutes (e.g. five minutes) and/or up to several hours (e.g. 6 hours or 12 hours).

Advantageously, soaking the feed powder in a solution containing the blowing agent(s) may degrade or attack the surface of the feed powder particles, thereby making the particles "sticky". Consequently, the blowing agent(s) may stick to the surface of the feed powder particles. Hence, the interaction between the blowing agent(s) and the powder particles as the spheroidisation flame is applied to the mixture may be improved.

**In** an embodiment, an agent may be utilised to make the surface of the feed powder particles "sticky" for the blowing agent(s). An example of a suitable agent is water soluble cellulose or a weak acid.

Advantageously, bubbles of gas generated by the blowing agent(s) may form more pores and/or generally larger pores in the powder particles, if the blowing agent(s) were stuck to the surface of the powder particles, e.g. following soaking of the feed powder in a solution containing the blowing agent(s) or coating of the feed powder with the blowing agent(s).

Acceptable porosity characteristics may also be realised without soaking the feed powder in a solution containing the blowing agent(s) or coating the feed powder with the blowing agent(s).

In an embodiment, the method may comprise a washing step to remove residual blowing agent(s). Typically, the washing step may be carried out after the step of applying the spheroidisation flame.

Advantageously, the washing step may also help to control porosity of the microspheres. The washing step may help to increase the size of surface pores and/or may enhance interconnected porosity.

The washing step may comprise washing the microspheres in an acidic solution. The acidic solution may comprise, for example, acetic acid.

The washing step may comprise soaking the microspheres in a fluid, e.g. an acidic solution. Further control of porosity, e.g. pore size, may be achieved by varying the length of time the microspheres are left to soak in the fluid. When an acidic solution is used, further control of porosity, e.g. pore size or interconnected porosity, may be achieved by varying the concentration of the acidic solution.

A second aspect of the invention provides a method of manufacture of a powder comprising, or consisting essentially of, microspheres, the method comprising:
manufacturing a first powder according to the first aspect of the invention;
manufacturing at least one further powder according to the first aspect of the invention, wherein the at least one further powder contains particles having a different size distribution and/or a different porosity from the first powder; and
mixing the first powder and the at least one further powder together.

The first powder and the further powder(s) may be mixed together in any ratio. The resulting powder may have any desired proportion of particles with particular size distributions and/or porosities.

In order that the invention may be well understood, it will now be described by way of example only with reference to the accompanying drawings in which:
Figure 1 is a scanning electron microscope (SEM) image of a plurality of porous resorbable microspheres according to a first example embodiment of the invention;
Figure 2 is a higher magnification SEM image of some of the porous resorbable microspheres shown in Figure 1;
Figure 3 is an SEM image of one of the porous resorbable microspheres shown in Figure 1;
Figure 4 is an SEM image of one of the porous resorbable microspheres shown in Figure 1;
Figure 5 is an SEM image of a plurality of porous resorbable microspheres according to a second example embodiment of the invention;
Figure 6 is a higher magnification SEM image of some of the porous resorbable microspheres shown in Figure 5;
Figure 7 is an SEM image of one of the porous resorbable microspheres shown in Figure 5;
Figure 8 is an SEM image of a plurality of porous resorbable microspheres according to a third example embodiment of the invention;
Figure 9 is a higher magnification SEM image of some of the porous resorbable microspheres shown in Figure 8;
Figure 10 is an SEM image of one of the porous resorbable microspheres shown in Figure 8;
Figure 11 is an SEM image of a plurality of porous resorbable microspheres according to a fourth example embodiment of the invention;
Figure 12 is an SEM image of one of the porous resorbable microspheres shown in Figure 11;
Figure 13 is an SEM image of one of the porous resorbable microspheres shown in Figure 11;
Figure 14 is a bar chart showing the results of surface area analysis carried out on two example embodiments of porous resorbable microspheres according to the invention;
Figure 15 is an SEM image of a cross-section of a porous resorbable microsphere according to the invention;
Figure 16 is an SEM image of an example embodiment of acid washed porous microspheres according to the invention:
Figure 17 is an SEM image of a plurality of non-porous borosilicate microspheres;
Figure 18 is an energy dispersive x-ray (EDX) spectrum for the non-porous borosilicate microspheres shown in Figure 17;
Figure 19 is an SEM image of a porous borosilicate microsphere according to another example embodiment of the invention;
Figure 20 is an EDX spectrum for the porous borosilicate microspheres shown in Figure 19;
Figure 21 is an SEM image of a plurality of non-porous Bioglass^{®} microspheres;
Figure 22 is an EDX spectrum for the non-porous Bioglass^{®} microspheres shown in Figure 21;
Figure 23 is an SEM image of a plurality of porous Bioglass^{®} microspheres according to another example embodiment of the invention;
Figure 24 is an EDX spectrum for the porous Bioglass^{®} microspheres shown in Figure 23;
Figure 25 is an SEM image of a plurality of non-porous borate glass microspheres;
Figure 26 is an EDX spectrum for the non-porous borate glass microspheres shown in Figure 25;
Figure 27 is an SEM image of a plurality of porous borate glass microspheres according to another example embodiment of the invention;
Figure 28 is an EDX spectrum for the porous borate glass microspheres shown in Figure 27;
Figure 29 is an SEM image of a plurality of non-porous apatite wollastonite microspheres;
Figure 30 is an EDX spectrum for the non-porous apatite wollastonite microspheres shown in Figure 29;
Figure 31 is an SEM image of a plurality of porous apatite wollastonite microspheres according to another example embodiment of the invention;
Figure 32 is an EDX spectrum for the porous apatite wollastonite microspheres shown in Figure 31;
Figure 33 is an SEM image of a plurality of non-porous hydroxyapatite microspheres;
Figure 34 is an EDX spectrum for the non-porous hydroxyapatite microspheres shown in Figure 33;
Figure 35 is an SEM image of a hollow hydroxyapatite microsphere according to another example embodiment of the invention;
Figure 36 is an EDX spectrum for the hollow hydroxyapatite microsphere shown in Figure 35;
Figure 37 is an SEM image of a non-porous β-TCP microsphere;
Figure 38 is an EDX spectrum for the non-porous β-TCP microsphere shown in Figure 37;
Figure 39 shows a space filled with porous resorbable microspheres according to the invention;
Figure 40 shows six images of porous calcium phosphate microspheres according to the invention loaded with human mesenchymal stem cells;
Figure 41 shows an experiment in which a dye/water solution is passed over irregularly-shaped glass micro particles;
Figure 42 shows an experiment in which a dye/water solution is passed over bulk (i.e. non-porous) microspheres;
Figures 43, 44 and 45 show an experiment in which a dye/water solution is passed over porous microspheres according to the invention; and
Figure 46 shows a cross-section of a microsphere according to another example embodiment of the invention, the microsphere having surface porosity and a solid core.

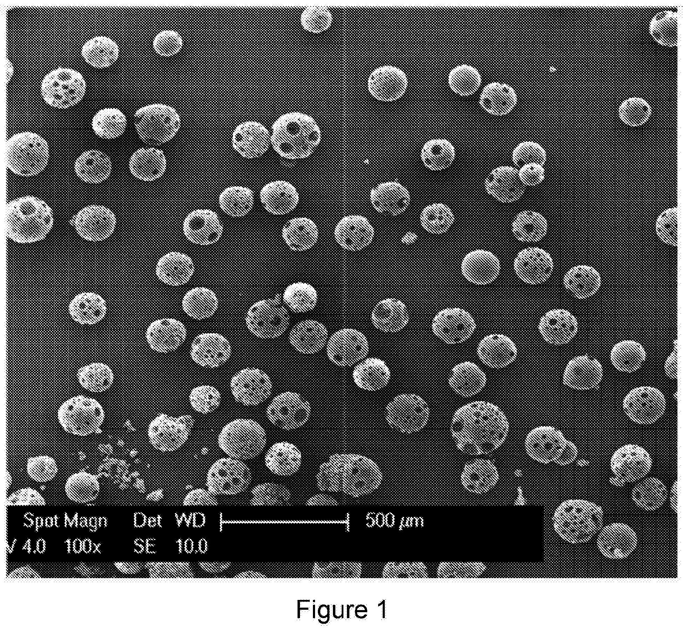

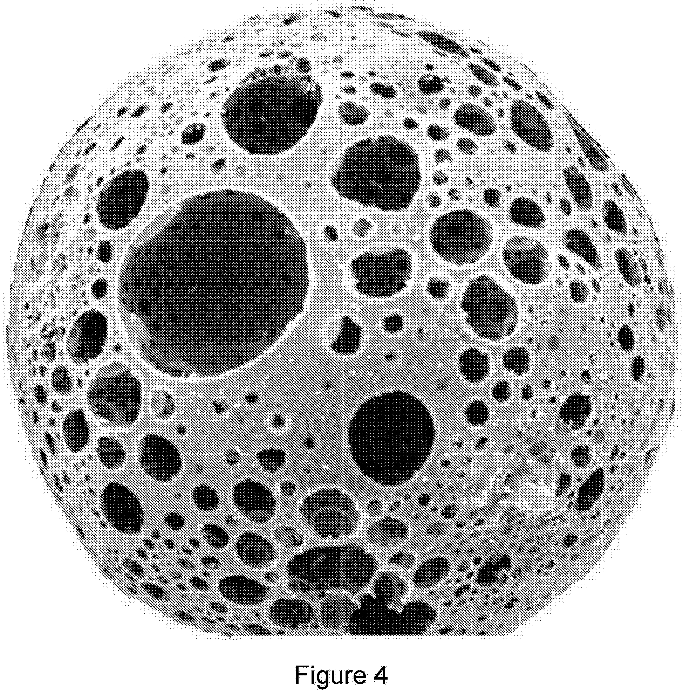

The resorbable porous microspheres shown in Figure 1, Figure 2, Figure 3 and Figure 4 were manufactured by flame-spraying spheroidisation. A feed powder comprising particles of calcium phosphate glass with a particle size of around 100 µm (± 40 µm) was soaked in a solution containing blowing agent(s) (e.g. calcium carbonate and/or sodium sulphate). After soaking, the mixture of the feed powder and the blowing agent(s) was supplied to a spray head and sprayed through a spheroidisation flame produced by an acetylene torch to produce the resorbable porous microspheres of the type shown in Figures 1, 2, 3 and 4. Carbon dioxide and/or sulphur dioxide generated from the blowing agent(s) create porosity within the calcium phosphate glass particles.

The resorbable porous microspheres shown in Figure 1 have a particle size of approximately 140 µm (± 50 µm). The yield of resorbable porous microspheres manufactured as described above was in excess of 95%. As can be seen from Figure 1, the resorbable porous microspheres have very good uniformity.

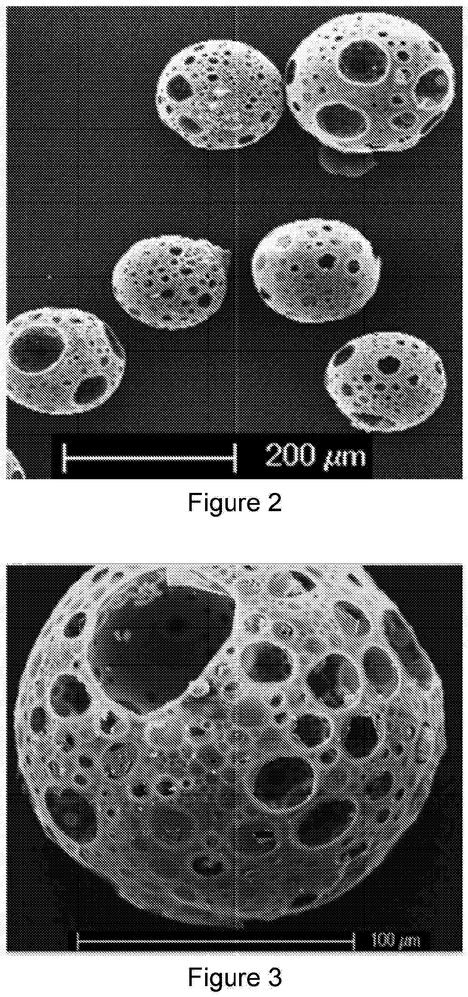

Figures 2, 3 and 4 are further images of the resorbable porous microspheres of Figure 1. Some areas of interconnected porosity can be seen in Figure 3.

The porosity of the resorbable porous microspheres of the type shown in Figures 1, 2, 3 and 4 was characterised using various techniques including mercury infusion porosimetry. A summary of the results is given in Table 1 below.

**Table 1**

| Average pore diameter (µm) | Bulk density (g/mL) | Apparent (skeletal) density (g/mL) | Open porosity (vol%) | Closed porosity (vol%) (estimated) | Total porosity (vol%) |
|---|---|---|---|---|---|
| 55 | 0.54 | 1.85 | 71 | 9 | 80 |

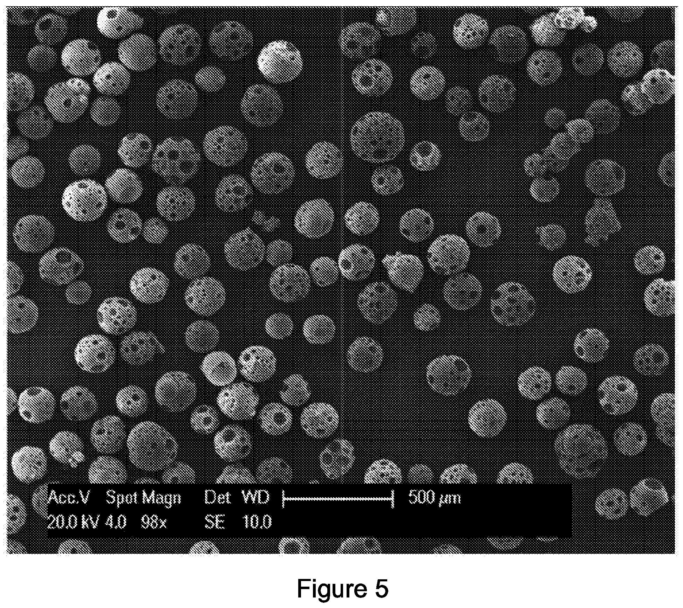

The resorbable porous microspheres shown in Figure 5, Figure 6 and Figure 7 were manufactured by flame-spraying spheroidisation. A feed powder comprising particles of calcium phosphate glass with a particle size of around 100 µm (± 40 µm) was soaked in a solution containing blowing agent(s) (e.g. calcium carbonate and/or sodium sulphate). After soaking, the mixture of the feed powder and the blowing agent(s) was supplied to a spray head and sprayed through a spheroidisation flame produced by an acetylene torch to produce the resorbable porous microspheres of the type shown in Figures 5, 6 and 7. Carbon dioxide and/or sulphur dioxide generated from the blowing agent(s) create porosity within the calcium phosphate glass particles.

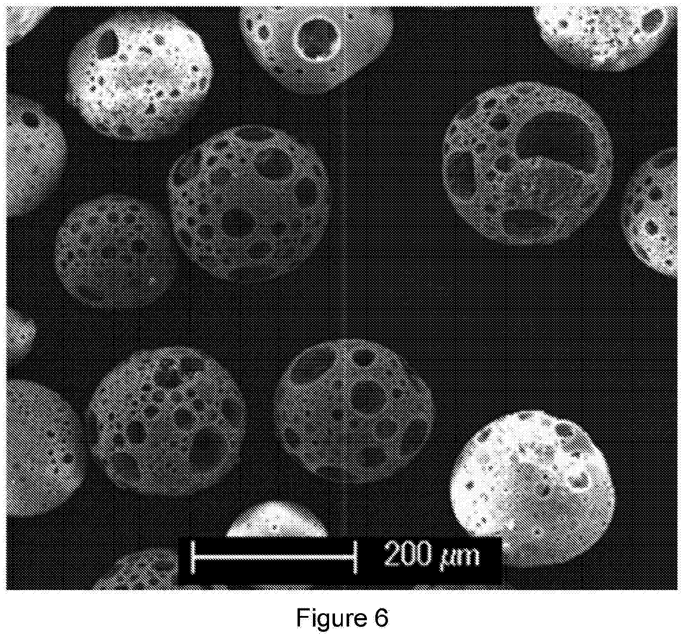

The resorbable porous microspheres shown in Figure 5, Figure 6 and Figure 7 have a particle size of approximately 140 µm (± 50 µm). The yield of resorbable porous microspheres manufactured as described above was in excess of 95%. As can be seen from Figure 5 and Figure 6, the resorbable porous microspheres have very good uniformity.

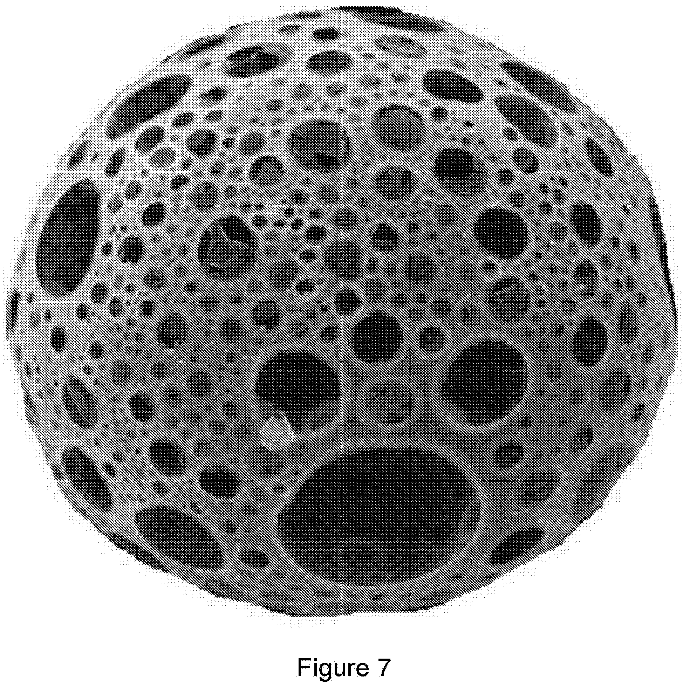

Figures 6 and 7 are further images of the resorbable porous microspheres of Figure 5.

The porosity of the resorbable porous microspheres of the type shown in Figures 5, 6 and 7 was characterised using various techniques including mercury infusion porosimetry. A summary of the results is given in Table 2 below.

**Table 2**

| Average pore diameter (µm) | Bulk density (g/mL) | Apparent (skeletal) density (g/mL) | Open porosity (vol%) | Closed porosity (vol%) (estimated) | Total porosity (vol%) |
|---|---|---|---|---|---|
| 56 | 0.58 | 1.66 | 65 | 14 | 79 |

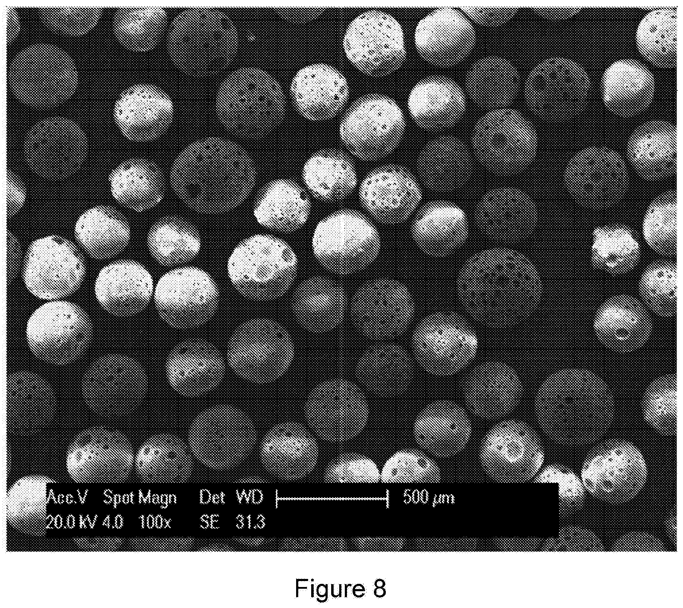

The resorbable porous microspheres shown in Figure 8, Figure 9 and Figure 10 were manufactured by flame-spraying spheroidisation. A feed powder comprising particles of calcium phosphate glass was soaked in a solution containing blowing agent(s) (e.g. calcium carbonate and/or sodium sulphate). After soaking, the mixture of the feed powder and the blowing agent(s) was supplied to a spray head and sprayed through a spheroidisation flame produced by an acetylene torch to produce the resorbable porous microspheres of the type shown in Figures 8, 9 and 10. Carbon dioxide and/or sulphur dioxide generated from the blowing agent(s) create porosity within the calcium phosphate glass particles.

The resorbable porous microspheres shown in Figure 8, Figure 9 and Figure 10 have an average particle size of approximately 300 µm. As can be seen from Figures 8, 9 and 10, the larger pores typically have a diameter of from 30 µm to 40 µm.

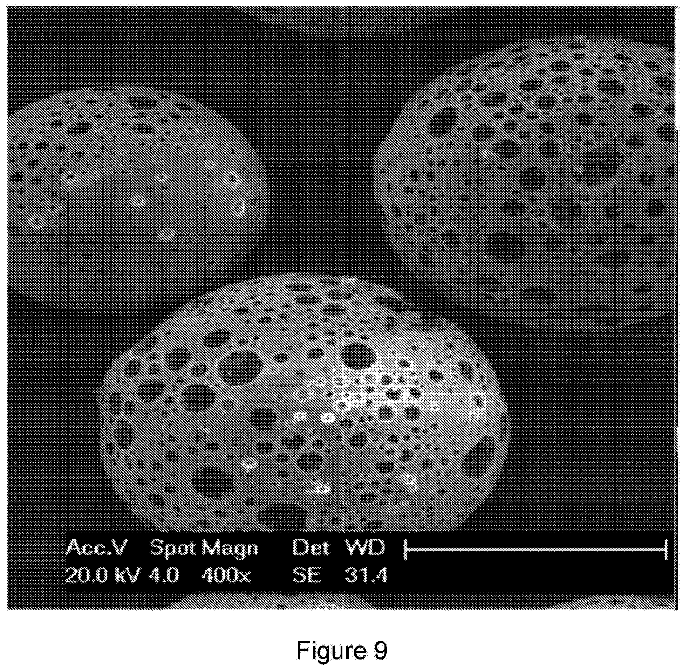

The yield of resorbable porous microspheres manufactured as described above was in excess of 95%. As can be seen from Figure 8 and Figure 9, the resorbable porous microspheres have very good uniformity.

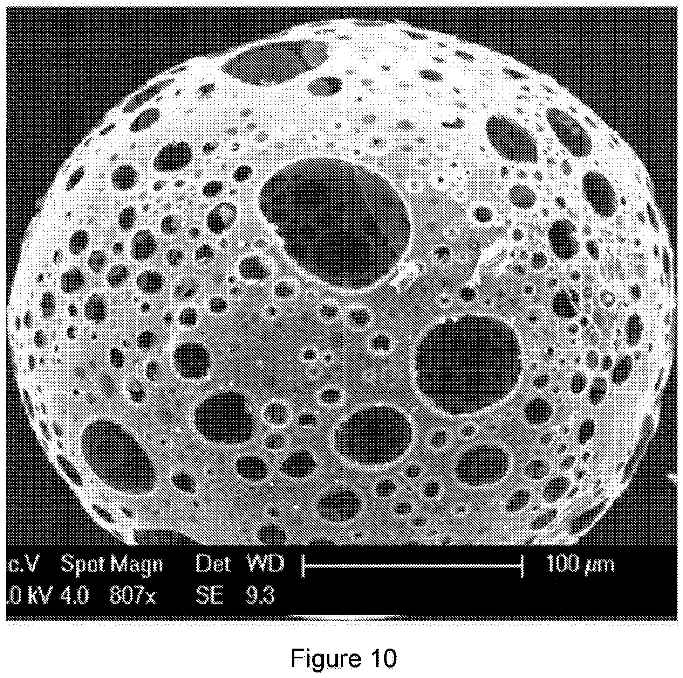

Figures 9 and 10 are further images of the resorbable porous microspheres of Figure 8.

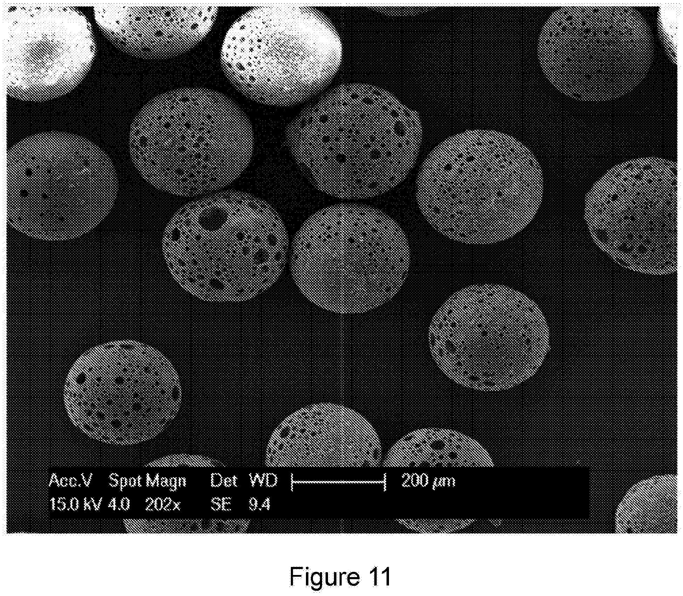

The resorbable porous microspheres shown in Figure 11, Figure 12 and Figure 13 were manufactured by flame-spraying spheroidisation. A feed powder comprising particles of calcium phosphate glass was soaked in a solution containing blowing agent(s) (e.g. calcium carbonate and/or sodium sulphate). After soaking, the mixture of the feed powder and the blowing agent(s) was supplied to a spray head and sprayed through a spheroidisation flame produced by an acetylene torch to produce the resorbable porous microspheres of the type shown in Figures 11, 12 and 13. Carbon dioxide and/or sulphur dioxide generated from the blowing agent(s) create porosity within the calcium phosphate glass particles.

The resorbable porous microspheres shown in Figure 11, Figure 12 and Figure 13 have an average particle size of approximately 300 µm. As can be seen from Figures 11, 12 and 13, the larger pores typically have a diameter of from 30 µm to 40 µm.

The yield of resorbable porous microspheres manufactured as described above was in excess of 95%. As can be seen from Figure 11, the resorbable porous microspheres have very good uniformity.

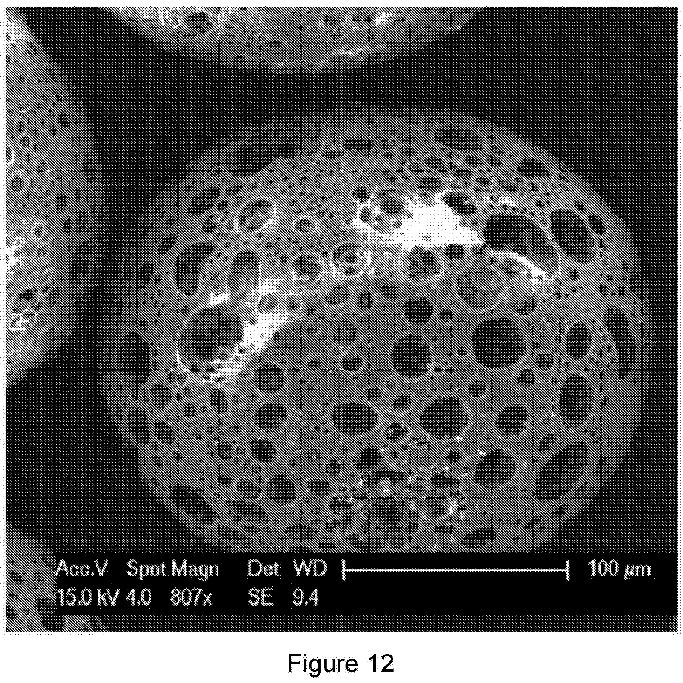

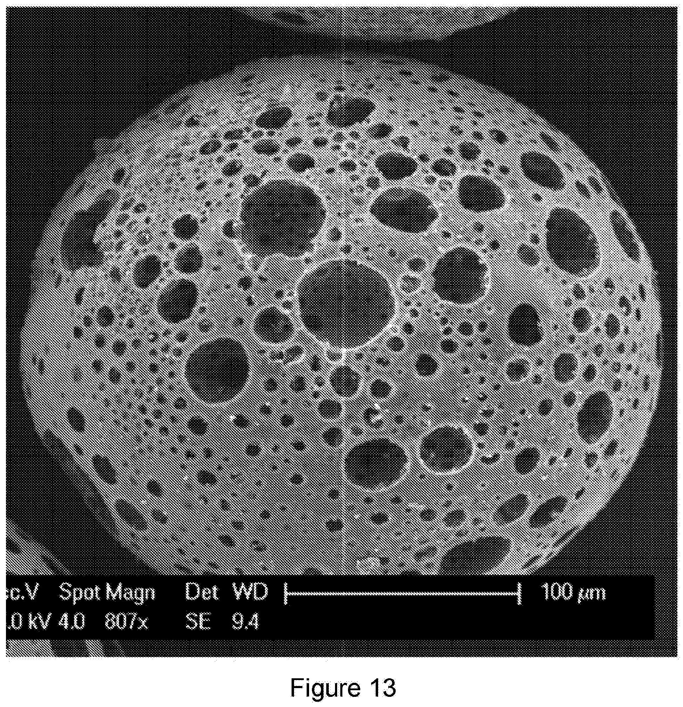

Figures 12 and 13 are further images of the resorbable porous microspheres of Figure 11.

Remnants of the blowing agent(s) used in the manufacture of porous resorbable microspheres according to the invention may be incorporated in the microspheres themselves. For instance, energy dispersive x-ray (EDX) analysis of a sample of porous resorbable microspheres according to the invention detected strontium within the microsphere composition, the strontium having come from the blowing agent, strontium carbonate, used in the manufacture of the microspheres. In some embodiments, the blowing agent(s) may be selected, in order to vary and/or finely control doping of the microsphere composition.

Advantageously, the methods of manufacture of the present invention may provide improved yields and/or uniformity of porous resorbable microspheres.

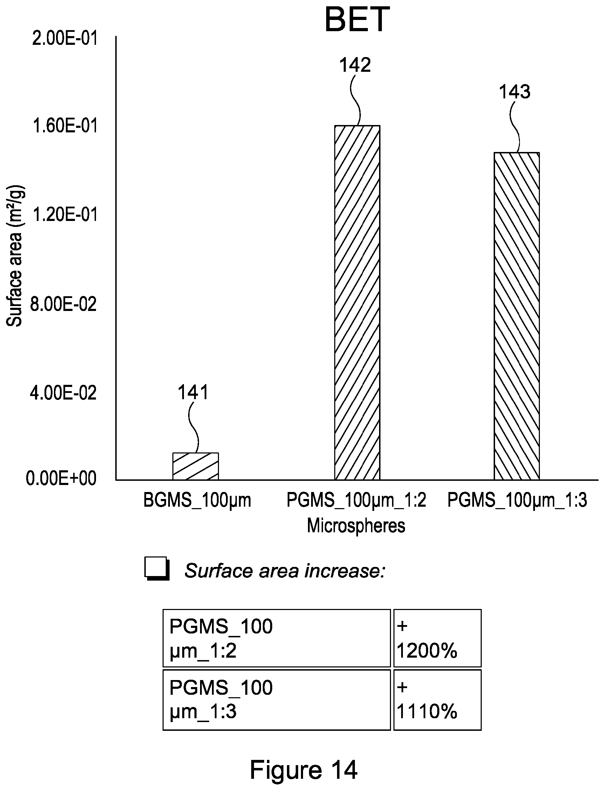

Figure 14 is a bar chart showing the results of a Brunauer, Emmett and Teller (BET) analysis of the specific surface area (surface area per unit mass) of two example embodiments of porous resorbable microspheres according to the invention compared with the particles of the bulk, substantially non-porous calcium phosphate glass feed powder used in the manufacture of the porous resorbable microspheres.

As indicated by a first column 141, the specific surface area of the bulk, substantially non-porous calcium phosphate glass feed powder was found to be around 0.01 m²/g. As indicated by a second column 142, the specific surface area of porous resorbable microspheres of the type shown in Figures 1, 2, 3 and 4 and discussed above was found to be around 0.16 m²/g. As indicated by a third column 143, the specific surface area of porous resorbable microspheres of the type shown in Figures 5, 6 and 7 and discussed above was found to be around 0.15 m²/g. The increase in specific surface area that occurs during manufacture of the porous resorbable microspheres from the bulk feed powder (column 141) is around 1200% for the porous resorbable microspheres of column 142 (i.e. porous resorbable microspheres of the type shown in Figures 1, 2, 3 and 4 and discussed above) and around 1110% for the porous resorbable microspheres of column 143 (i.e. porous resorbable microspheres of the type shown in Figures 5, 6 and 7 and discussed above)

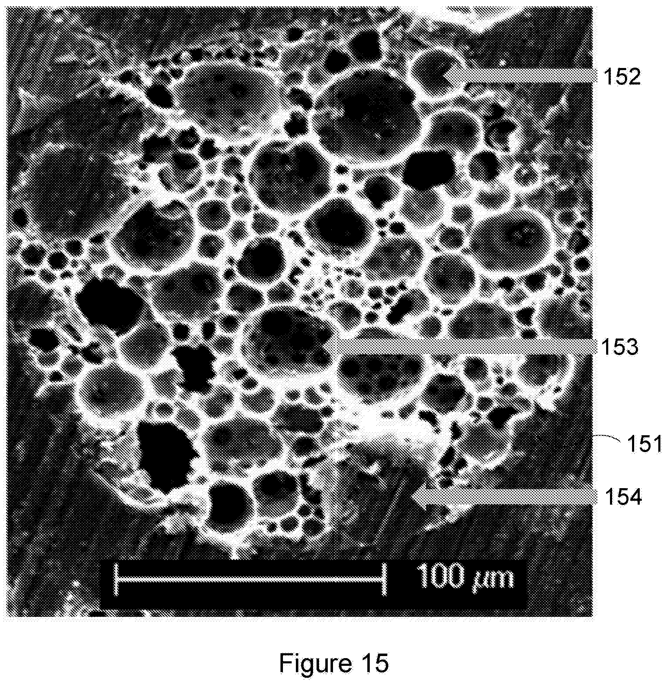

Figure 15 is an SEM image of a cross-section through a porous resorbable microsphere 151 according to the invention. The porous structure of the porous resorbable microsphere 151 can be seen clearly in Figure 15. The porous resorbable microsphere 151 contains closed pores, interconnected pores and open, surface pores. An example of a closed pore is labelled 152, an example of an interconnected pore is labelled 153 and an example of an open, surface pore is labelled 154.

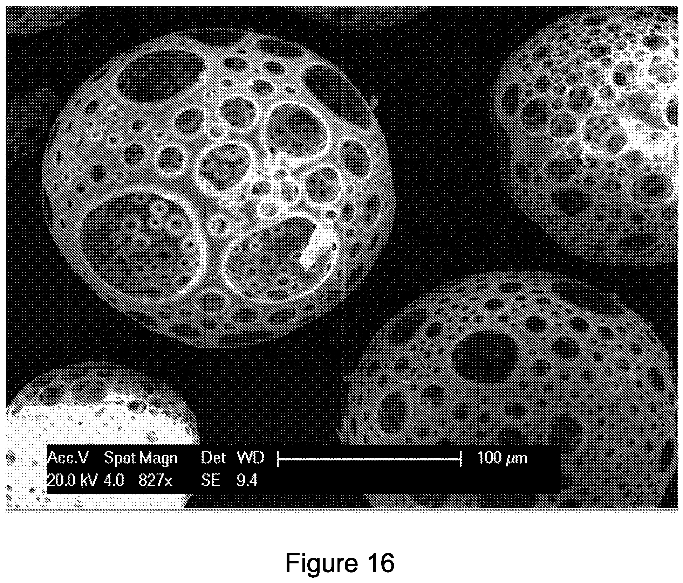

Figure 16 shows a plurality of acid-washed porous calcium phosphate glass microspheres according to another example embodiment of the invention. The microspheres have a diameter of around 100 µm. After the spheroidisation flame had been applied, the calcium phosphate glass microspheres were washed using an acidic solution comprising acetic acid. As can be seen from Figure 16, the microspheres have relatively larger surface pores and also have a relatively high amount of interconnected porosity.

The data presented in Table 3 below illustrate the effect of washing the microspheres in an acetic acid solution. Two types of microspheres according to the invention were washed in an acetic acid solution. The first type of microspheres (A) were calcium phosphate glass microspheres, having a diameter of approximately 100 µm. The second type of microspheres (B) were calcium phosphate glass microspheres, having a diameter of approximately 100 µm. During manufacture, the ratio by weight of the blowing agent(s) to the calcium phosphate glass particles was different for the two types of microspheres (A and B).

The open porosity of the microspheres was measured pre- and post-wash. The closed porosity of the microspheres was measured pre- and post-wash. Hence, the total porosity of the microspheres could be calculated pre- and post-wash.

For the first type of microspheres (A), washing led to a slight increase in total porosity. Slight increases in the open porosity and/or the closed porosity contributed to the slight increase in total porosity.

For the second type of microspheres (B), washing resulted in a slightly larger increase in total porosity than for the first type of microspheres (A). The increase in total porosity of the second type of microspheres (B) arose, due to a large increase in open porosity, which was offset to some extent by a decrease in closed porosity.

**Table 3**

| Microspheres | Open porosity (vol%) | | Closed porosity (vol%) | | Total porosity (vol%) | |
|---|---|---|---|---|---|---|
| | Pre-acid wash | Post-acid wash | Pre-acid wash | Post-acid wash | Pre-acid wash | Post-acid wash |
| A | 71 | 72 (±2) | 9 | 10 (±3) | 80 | 82 (±1) |
| B | 65 | 76 (±2) | 14 | 7 (±3) | 79 | 83 (±2) |

Without wishing to be bound by any theory, it is thought that washing the microspheres in acetic acid solution removes residual blowing agent(s) from the microspheres. The removal of residual blowing agent(s) may contribute at least partially to an increase in total porosity of the microspheres. Pores that were closed or obstructed due to the presence of residual blowing agent(s) may be opened as a result of the washing.

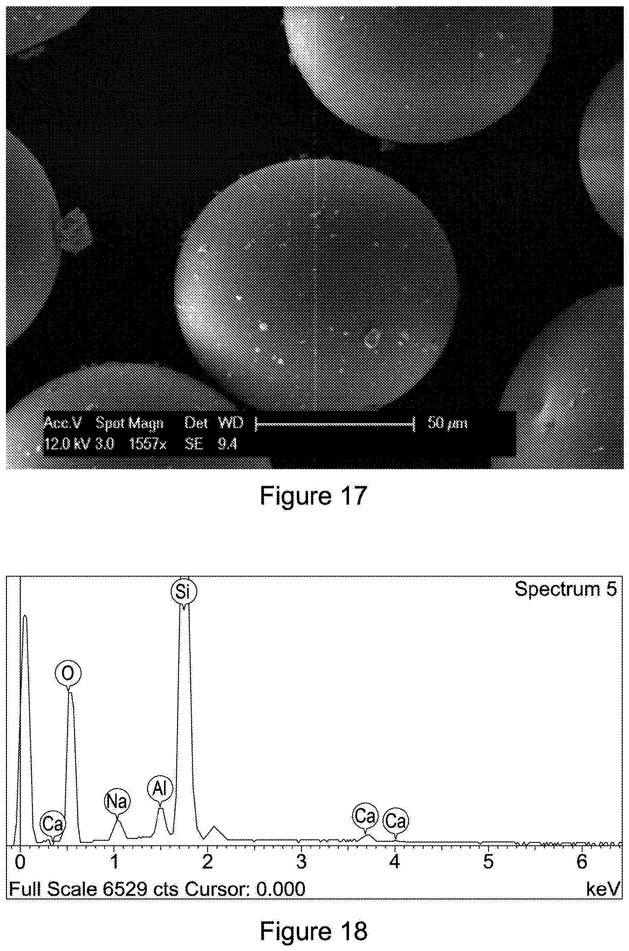

Figure 17 is an SEM image of a plurality of non-porous borosilicate microspheres. The non-porous borosilicate microspheres were manufactured by flame spraying spheroidisation using an acetylene torch. The non-porous borosilicate microspheres have a diameter of approximately 85 µm and have very good sphericity.

Figure 18 is an EDX spectrum for the non-porous borosilicate microspheres shown in Figure 17.

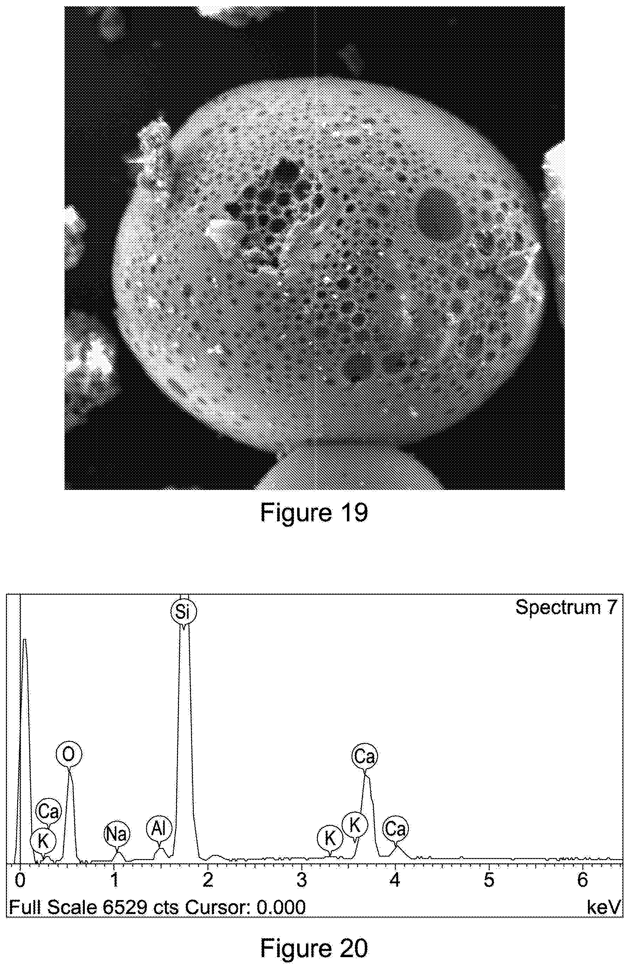

Figure 19 is an SEM image of a porous borosilicate microsphere according to another example embodiment of the invention. The porous borosilicate microspheres were manufactured by flame spraying spheroidisation using an acetylene torch. The porous borosilicate microspheres have good sphericity.

Figure 20 is an EDX spectrum for the porous borosilicate microspheres shown in Figure 19.

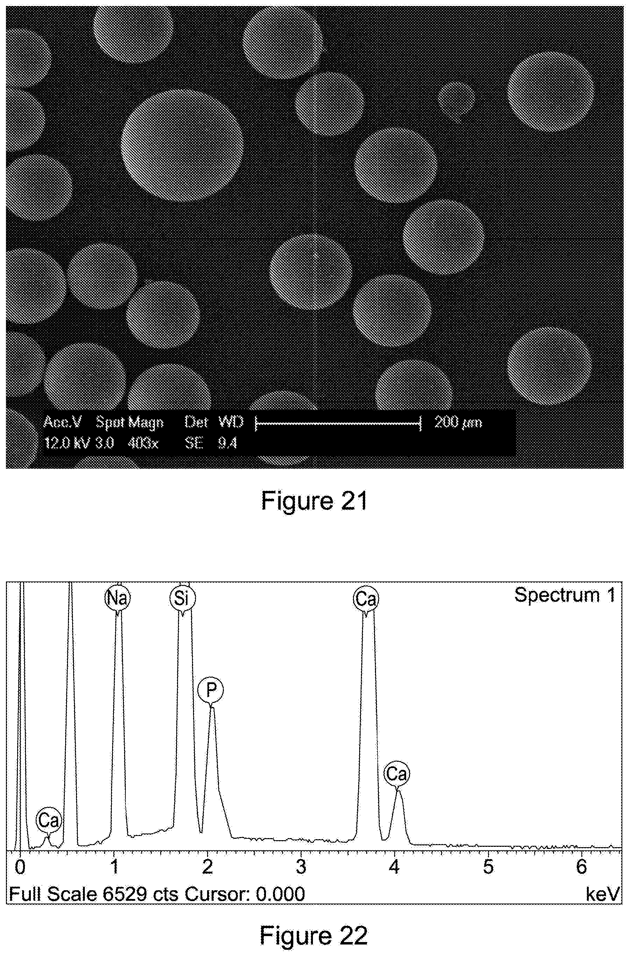

Figure 21 is an SEM image of a plurality of non-porous Bioglass^{®} microspheres. The non-porous Bioglass^{®} microspheres were manufactured by flame spraying spheroidisation using an acetylene torch. The non-porous Bioglass^{®} microspheres have very good sphericity. The non-porous Bioglass^{®} microspheres have a range of diameters, from approximately 40 µm to approximately 200 µm. A significant number of the non-porous Bioglass^{®} microspheres shown in Figure 21 have a diameter of approximately 110 µm, while another significant number of the Bioglass^{®} microspheres shown in Figure 21 have a diameter of approximately 120 µm.

Figure 22 is an EDX spectrum for the non-porous Bioglass^{®} microspheres shown in Figure 21.

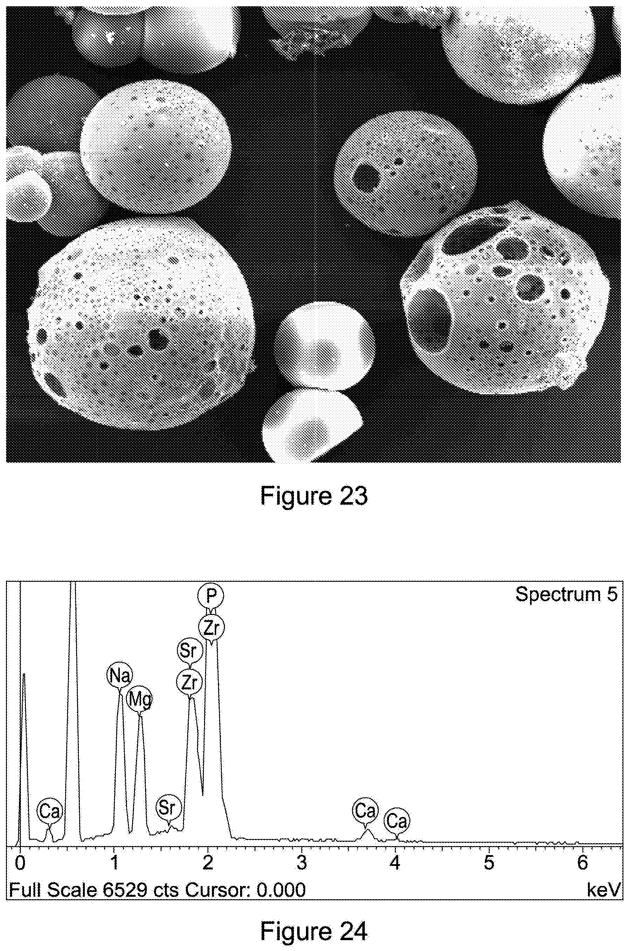

Figure 23 is an SEM image of a plurality of porous Bioglass^{®} microspheres according to another example embodiment of the invention. The porous Bioglass^{®} microspheres were manufactured by flame spraying spheroidisation using an acetylene torch. The porous Bioglass^{®} microspheres have good sphericity.

Figure 24 is an EDX spectrum for the porous Bioglass^{®} microspheres shown in Figure 23. The Zr in the EDX spectrum may be from contamination during grinding in a ball mill. The appearance of Sr in the EDX spectrum was surprising and unexpected; it could be due to overlap in Ca/Sr peaks or contamination.

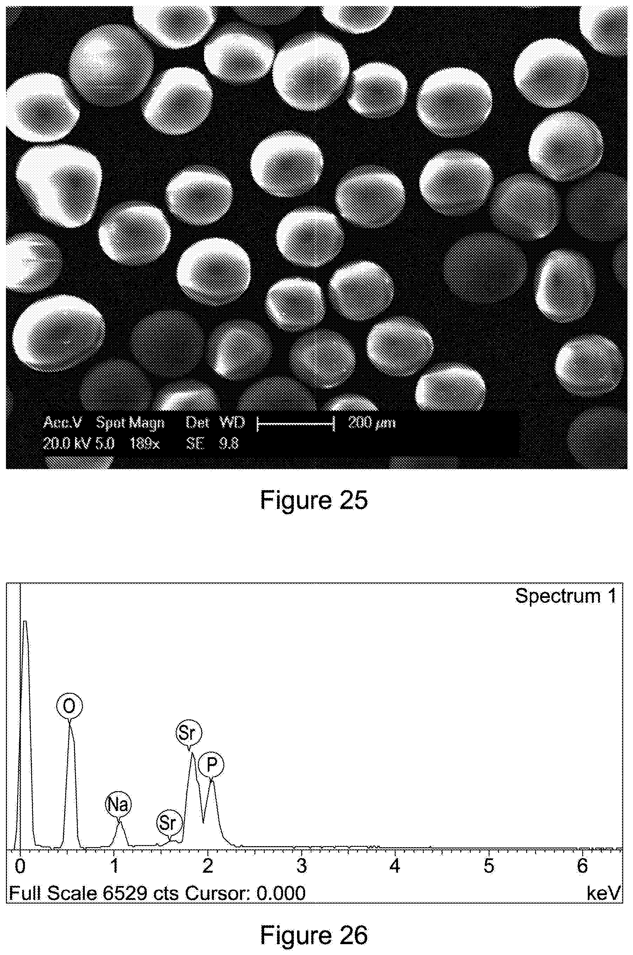

Figure 25 is an SEM image of a plurality of non-porous borate glass microspheres. The non-porous borate glass microspheres were manufactured by flame spraying spheroidisation using an acetylene torch. The non-porous borate glass microspheres have very good sphericity. The non-porous borate glass microspheres have diameters of from approximately 150 µm to approximately 250 µm.

Figure 26 is an EDX spectrum for the non-porous borate glass microspheres shown in Figure 25.

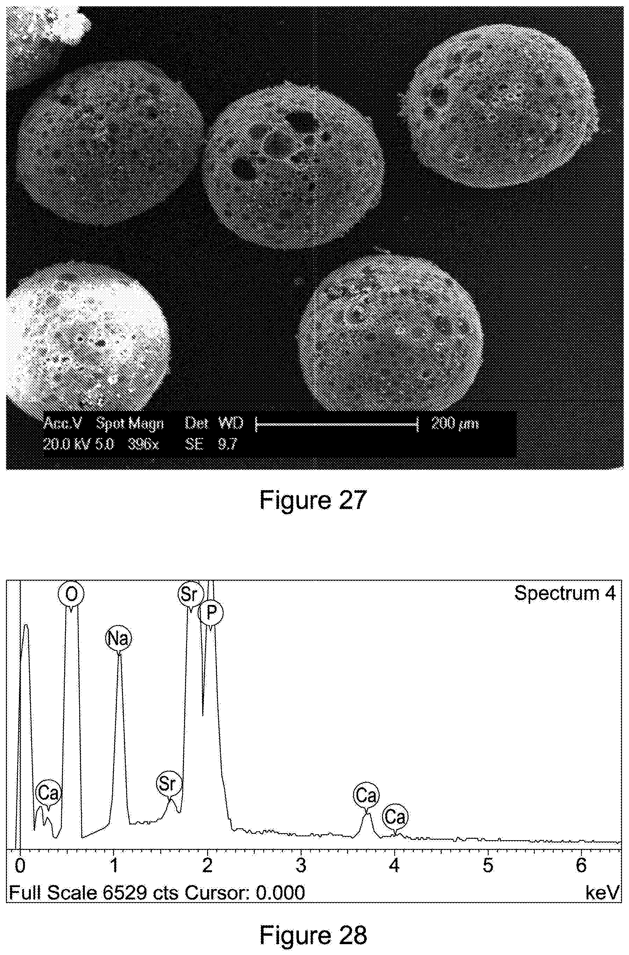

Figure 27 is an SEM image of a plurality of porous borate glass microspheres according to another example embodiment of the invention. The porous borate glass microspheres were manufactured by flame spraying spheroidisation using an acetylene torch. The porous borate glass microspheres have good sphericity. The porous borate glass microspheres have diameters of from approximately 220 µm to approximately 250 µm.

Figure 28 is an EDX spectrum for the non-porous borate glass microspheres shown in Figure 27.

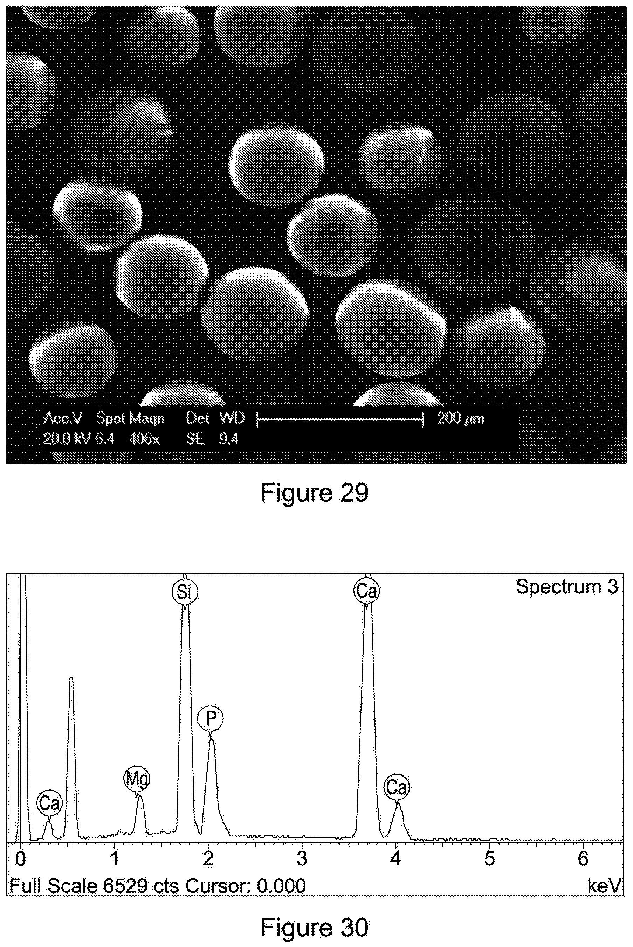

Figure 29 is an SEM image of a plurality of non-porous apatite wollastonite microspheres. The non-porous apatite wollastonite microspheres were manufactured by flame spraying spheroidisation using an acetylene torch. The non-porous apatite wollastonite microspheres have good sphericity. The non-porous apatite wollastonite microspheres have a range of diameters, from approximately 100 µm to approximately 140 µm.

Figure 30 is an EDX spectrum for the non-porous apatite wollastonite microspheres shown in Figure 29.

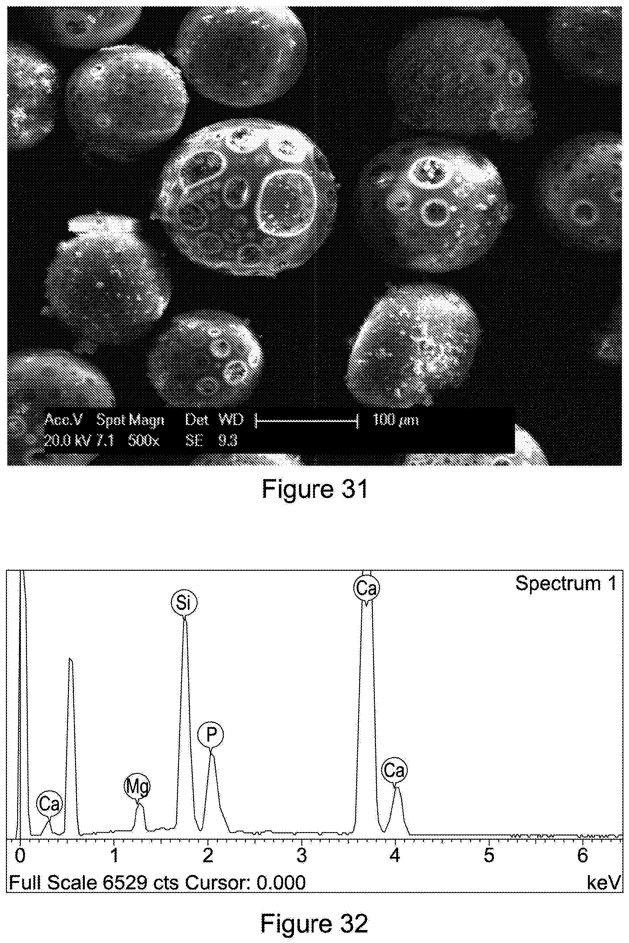

Figure 31 is an SEM image of a plurality of porous apatite wollastonite microspheres according to another example embodiment of the invention. The porous apatite wollastonite microspheres were manufactured by flame spraying spheroidisation using an acetylene torch. The porous apatite wollastonite microspheres have good sphericity. The porous apatite wollastonite microspheres have a range of diameters, from approximately 100 µm to approximately 170 µm.

Figure 32 is an EDX spectrum for the non-porous apatite wollastonite microspheres shown in Figure 31.

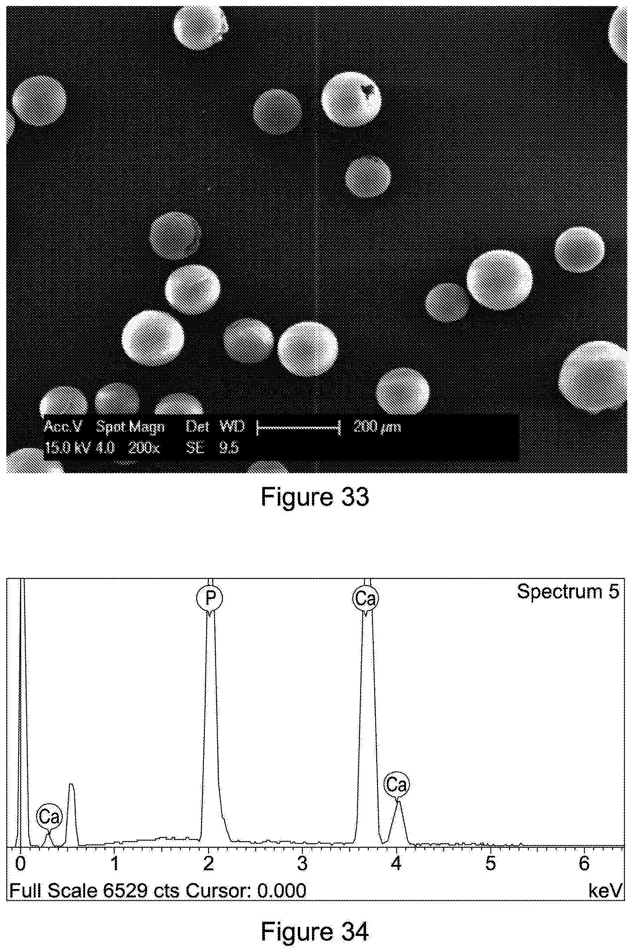

Figure 33 is an SEM image of a plurality of non-porous hydroxyapatite microspheres. The non-porous hydroxyapatite microspheres were manufactured by flame spraying spheroidisation using an acetylene torch. The non-porous hydroxyapatite microspheres have good sphericity. The non-porous hydroxyapatite microspheres have a range of diameters, from approximately 100 µm to approximately 160 µm.

Figure 34 is an EDX spectrum for the non-porous hydroxyapatite microspheres shown in Figure 33.

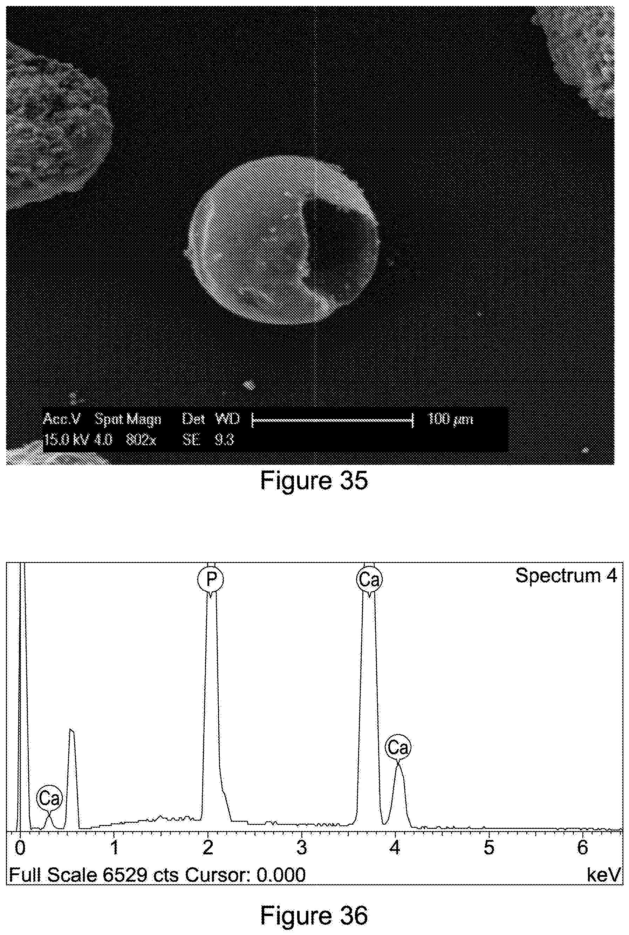

Figure 35 is an SEM image of a hollow hydroxyapatite microsphere according to another example embodiment of the invention. The hollow hydroxyapatite microsphere was manufactured by flame spraying spheroidisation using an acetylene torch. The hollow hydroxyapatite microsphere has good sphericity. The hollow hydroxyapatite microsphere has a diameter of approximately 120 µm.

Figure 36 is an EDX spectrum for the hollow hydroxyapatite microsphere shown in Figure 35.

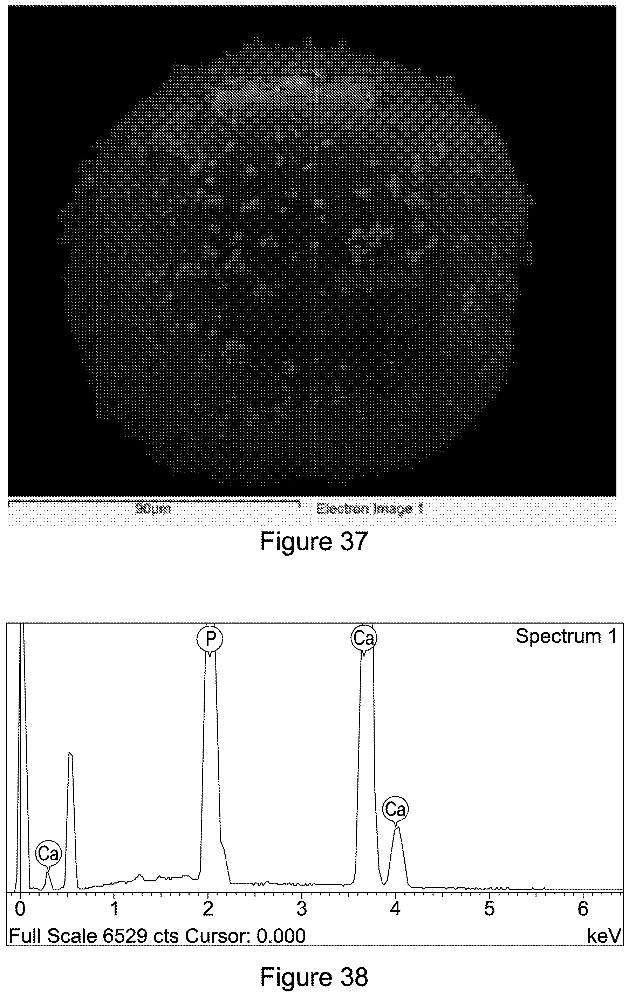

Figure 37 is an SEM image of a non-porous β-TCP microsphere. The non-porous β-TCP microsphere was manufactured by flame spraying spheroidisation using an acetylene torch. The non-porous β-TCP microsphere has good sphericity. The non-porous β-TCP microsphere has a diameter of approximately 150 µm.

Figure 38 is an EDX spectrum for the non-porous β-TCP microspheres shown in Figure 37.

A better packing efficiency may be achieved by providing microspheres of a plurality of different sizes.

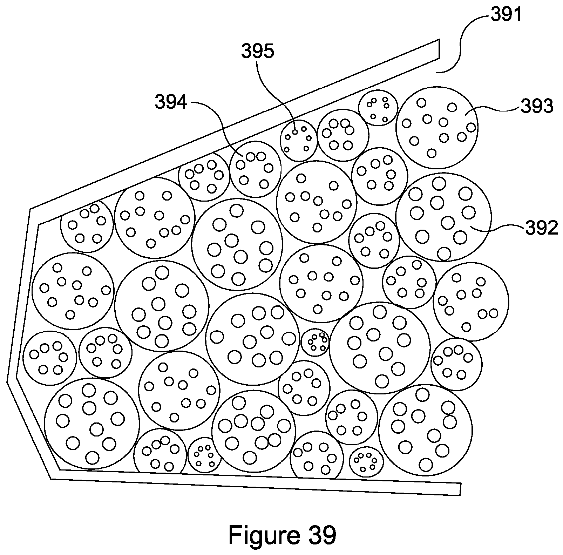

Figure 39 shows an irregularly-shaped space 391 filled with a powder comprising resorbable porous microspheres according to the invention. The powder comprises four different, distinctly-sized, types of resorbable porous microspheres according to the invention. A first type of resorbable porous microsphere 392 has a larger diameter than a second type of resorbable porous microsphere 393, which has a larger diameter than a third type of resorbable porous microsphere 394, which has a larger diameter than a fourth type of resorbable porous microsphere 395.

A powder comprising resorbable porous microspheres according to the invention may contain any number of types of porous resorbable microspheres mixed in any ratio. Accordingly, a powder may be produced having particles of more than one distinct particle size distribution and/or porosity.

The powder may have any particle size distribution. For instance, the powder may have a monomodal, bimodal, trimodal, tetramodal, pentamodal or hexamodal particle size distribution. Different particle size distributions may be better suited for different applications.

The irregularly-shaped space could be, for example, a space between two sections of bone or a defect or void within a bone.

One application for resorbable porous microspheres of the invention is in bone tissue regeneration, e.g. in the treatment of osteoporosis or other bone resorption disorders. For this application, calcium phosphate microspheres according to the invention may be loaded with autologous stem cells (or other cell types) and/or other biological components. The resorbable porous microspheres of the invention could be used as a bone graft substitute.

Osteoporosis and fragility fractures are a major problem worldwide, particularly in countries with aging populations. As a consequence, there is an ever-growing need for long-term orthopaedic care.

The present invention may help to facilitate a shift from tissue repair to tissue regeneration. By facilitating a shift from tissue repair to tissue regeneration, the growth rate of the need for long-term orthopaedic care may be reduced.

Across Europe, an estimated four million new fractures occur per year (around eight fractures each minute or one every eight seconds). The total direct cost of these fractures has been estimated at €31.7 billion, which is forecast to increase to €76.7 billion by 2050 based on anticipated changes in the demography of Europe.

**In** the UK, the annual combined healthcare and social cost for fractures in bones weakened by osteoporosis is nearly £1.73 billion.

In the UK, currently nearly 20 million people are aged 50 or more. This is predicted to increase to 25 million by 2020. Over 60,000 hip, 50,000 wrist and 120,000 vertebral osteoporosis-related fractures occur each year in the UK. According to the National Osteoporosis Society, recent trends suggest that hip fracture rates will increase to 117,000 by 2016.

In 2001, combined NHS and social care costs for a single hip fracture in the UK were estimated to be £20000.

Each year fractures in patients aged 60 and over account for more than two million hospital bed days in England alone. Around 30% of over 65 year olds living in the community will fall each year (increasing to 42% for the over 75 age group), while over 60% of people in care homes fall each year.

Usually, treatment is not administered until after a person, e.g. an elderly person with osteoporosis, has suffered a broken bone. Advantageously, treatment using the present invention may be administered prior to any fractures (or any further fractures) occurring, in order to reduce the likelihood of an individual suffering a fracture in a bone weakened by osteoporosis. Apart from patient benefits, this may also lead to significant social and healthcare cost savings.

In an example embodiment of the invention, an individual may be identified as having or being at risk of having osteoporosis. For instance, the individual may have suffered (or be at risk of suffering) a fracture, e.g. an osteoporotic compression fracture. The individual may then have an examination, typically an x-ray examination, in order to identify any regions of resorbed osteoporotic bone. The examination, e.g. the x-ray examination, may comprise a whole-body scan. A whole-body scan may be able to provide information on overall and local bone mineral content (BMC) and bone mineral density (BMD).

Autologous stem cells may then be isolated from the individual. The autologous stem cells isolated from the individual are then loaded into bio-resorbable porous microspheres according to the invention.

The bio-resorbable porous microspheres loaded with the autologous stem cells may then be injected into the identified region(s) of resorbed osteoporotic bone. Typically, this may involve only a minimally invasive surgical procedure using needles or cannulae. Accordingly, the individual may be treated as a hospital day-case patient.

The bio-resorbable porous microspheres may dissolve over time within the body, without causing any harm to the individual. The autologous stem cells will act to promote regeneration of bone tissue, thereby strengthening the identified region(s) of resorbed osteoporotic bone. Hence, the likelihood of the individual suffering a bone fracture may be reduced.

An individual may be found to have a region of resorbed osteoporotic bone. The region of resorbed osteoporotic bone could be in any part of the individual's skeleton, e.g. the spine, femur, hip, ankle or wrist. A syringe or cannula may be used to inject porous bio-resorbable microspheres loaded with autologous stem cells isolated from the individual into the region of resorbed osteoporotic bone.

It will be appreciated that the porous resorbable microspheres of the invention may provide an osteoporotic fracture prevention prophylactic. Advantageously, this preventative treatment may be delivered non-invasively or via a minimally invasive surgical procedure.

While dissolving within the body, the bio-resorbable porous microspheres may release active and/or therapeutic agents, e.g. ions, other than, or as well as, cells such as autologous stem cells.

The applicant has carried out experiments in which human mesenchymal stem cells (hMSC) have been loaded into porous calcium phosphate glass microspheres according to the invention. Porous resorbable microspheres comprising pores having larger diameters may be preferred for applications in which the porous resorbable microspheres are loaded with stem cells.

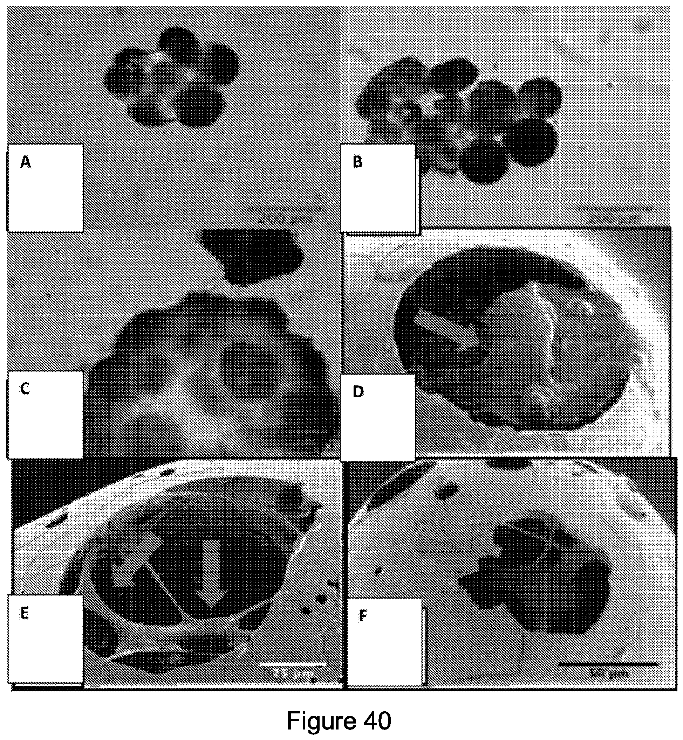

Figure 40 includes six images (labelled A, B, C, D, E and F) of calcium phosphate microspheres loaded with human mesenchymal stem cells (hMSC). Images A, B and C show an in vitro multicellular hMSC aggregate formation incorporating porous calcium phosphate microspheres. Images D, E and F are SEM images of human mesenchymal stem cells within the pores of the calcium phosphate microspheres. The arrows in images D, E and F point towards the human mesenchymal stem cells.

Porous microspheres according to the invention may be loaded with agents other than stem cells, e.g. cells, growth factors, proteins or pharmaceutically active agents.

The porous microspheres of the invention may have utility in the treatment of fractures, e.g. osteoporotic fractures such as osteoporotic vertebral fractures.

The porous microspheres of the invention may find utility as a bone graft material.

Application of the porous resorbable microspheres of the invention is not limited to bone regeneration.

The porous resorbable microspheres may be loaded with chemical or biological drugs or other active agents for release into the human or animal body.

The invention may also have utility in non-biomedical applications. An example of a non-biomedical application is filtration and separation.

For instance, porous microspheres may be used to separate a mixture of large and small molecules. An initial mixture of larger molecules and smaller molecules is fed to a gel filtration resin comprising a plurality of porous microspheres according to the invention. The smaller molecules may be "included" (i.e. be small enough to pass into and through the pores of porous microspheres), while the larger molecules may be "excluded" (i.e. be too large to enter the pores of the porous microspheres 203). Hence, the larger molecules 201 may be eluted before the smaller molecules 202.

Another potential use for porous resorbable microspheres according to the invention is as a replacement for "microbeads" which are found in many beauty and cleaning products.

Microbeads are typically made from plastic and are included in products such as shower gel, face washes, toothpaste and cleaning products for their abrasive qualities.

A problem with microbeads is that typically they may be too small to be filtered out at water treatment plants and consequently may end up in lakes and rivers. The plastic may soak up toxins and be eaten by fish and other creatures. **In** this way, there is a concern that toxins may build up in the food chain and eventually be consumed by humans.

Porous resorbable microspheres according to the present invention may be used as a substitute for microbeads. They could provide the required abrasive qualities before dissolving harmlessly into the environment, e.g. in a lake or river, in a fish or other creature or in a human or animal at the top of the food chain.

Other potential applications for microspheres according to the invention may include: use as a feedstock for additive manufacturing; filtration; separation; fluid, e.g. water, purification; beauty and personal care products such as cosmetics, shower gel and face wash; laundry and cleaning products; or use in applications requiring a lightweight low thermal expansion and low conductivity material.

For example, porous and/or non-porous microspheres, e.g. glass or glass-ceramic bulk (i.e. non-porous) and/or porous microspheres according to the invention may be used in combination with other particles or bodies such as microspheres. Such other particles or bodies may comprise polymer microspheres.

In an example embodiment, porous and/or non-porous microspheres according to the invention may be used in combination with natural or synthetic polymer microspheres. The utilisation of natural or synthetic polymer microspheres may help to achieve control over drug and/or biological component release. Additionally or alternatively, the natural or synthetic polymer microspheres may be utilised to deliver alternate drugs or biological components directly to sites of interest.

In another example embodiment, combining fast-resorbing microspheres, e.g. glass microspheres such as calcium phosphate microspheres, according to the invention with polymer microspheres could be used to achieve control over the polymer degradation profiles and vice versa - acidic release from polymer microspheres could be used to control release from the glass microspheres.

In another example embodiment, microspheres according to the invention, e.g. bulk (i.e. non-porous) and/or porous glass or glass-ceramic microspheres, may be coated at least partially with one or more resorbable (natural or synthetic) polymers to gain improved control over release of components carried by, e.g. encapsulated within and/or coated on, the microspheres.

Gaining control of particle geometry can be critical for additive manufacturing (e.g. 3D printing). Accordingly, microspheres according to the invention may be well suited for use in a feedstock for an additive manufacturing process, due to their uniformity and/or sphericity.

Currently, for instance, there is significant interest in possible additive manufacturing of biological materials or components, but it is proving extremely difficult to achieve and/or optimise. In an example embodiment, porous microspheres according to the invention may be loaded with one or more biological components (or non-biological entities) of interest to provide a feedstock. The feedstock may then be supplied to a 3D printer or other additive manufacturing device operable to produce a component having a desired geometry. With careful control over the composition of the microspheres, e.g. glass formulations of the microspheres, the component could then be engineered to degrade away (over time and/or in situ), leaving behind the incorporated biological components.

Microspheres according to the present invention may be utilised in separation and/or filtration applications.

In an example, it is envisaged that microspheres according to the invention may have utility in filtration devices within industrial applications (such as desalination plants), e.g. to remove heavy metals or bacteria.

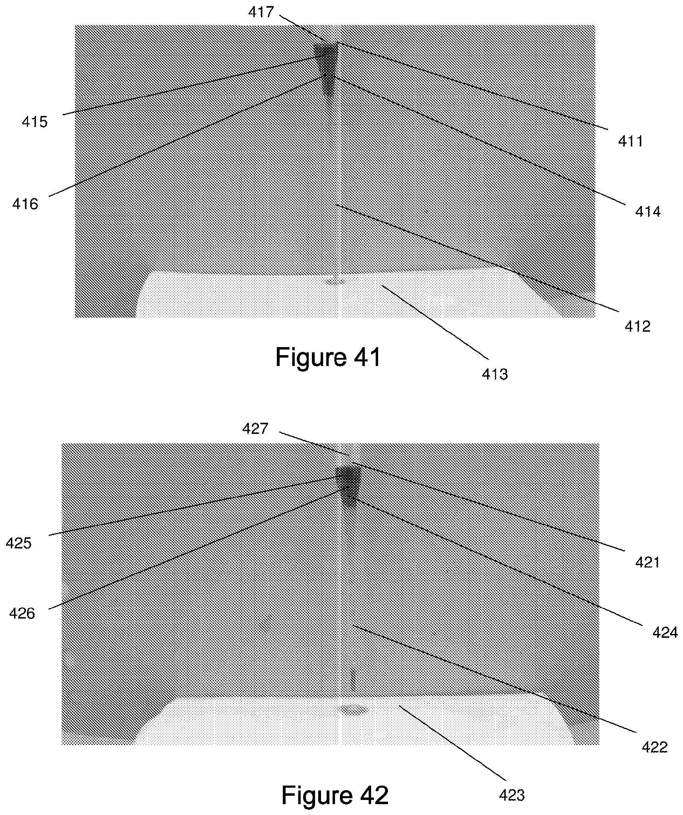

Figure 41 shows an experiment in which a dye/water solution 415 is passed over ground irregularly-shaped, non-porous glass micro-particles 414. A vertically-oriented glass micropipette 417 having an upper, wider portion 411 and a lower, thinner portion 412 is arranged above a blotting surface 413. A tapered portion 416 connects the upper, wider portion 411 to the lower, thinner portion 412. The irregularly-shaped glass micro-particles 414 are provided within the tapered portion 416. In the experiment, the dye/water solution 415 is passed through the micropipette 417. It took five minutes for the solution to pass through the micropipette 417 on to the blotting surface 413.

Figure 42 shows an experiment in which a dye/water solution 425 is passed over non-porous microspheres 424. A vertically-oriented glass micropipette 427 having an upper, wider portion 421 and a lower, thinner portion 422 is arranged above a blotting surface 423. A tapered portion 426 connects the upper, wider portion 421 to the lower, thinner portion 422. The non-porous microspheres according to the invention 424 are provided within the tapered portion 426. In the experiment, the dye/water solution 425 is passed through the micropipette 427. It took around 90 seconds for the solution to pass through the micropipette 427 on to the blotting surface 423.

The solution passed through the micropipette much quicker in the experiment shown in Figure 42 than in the experiment shown in Figure 41. Without wishing to be bound by any theory, it is postulated that the uniformity and sphericity of the non-porous microspheres according to the invention 424, as compared with the irregularly-shaped glass micro-particles 414, was responsible in large part for the significant increase in the flow rate of the dye/water solution through the micropipette.

It is noted that in both Figure 41 and Figure 42, the dye/water solution is not separated as it passes through the micropipette.

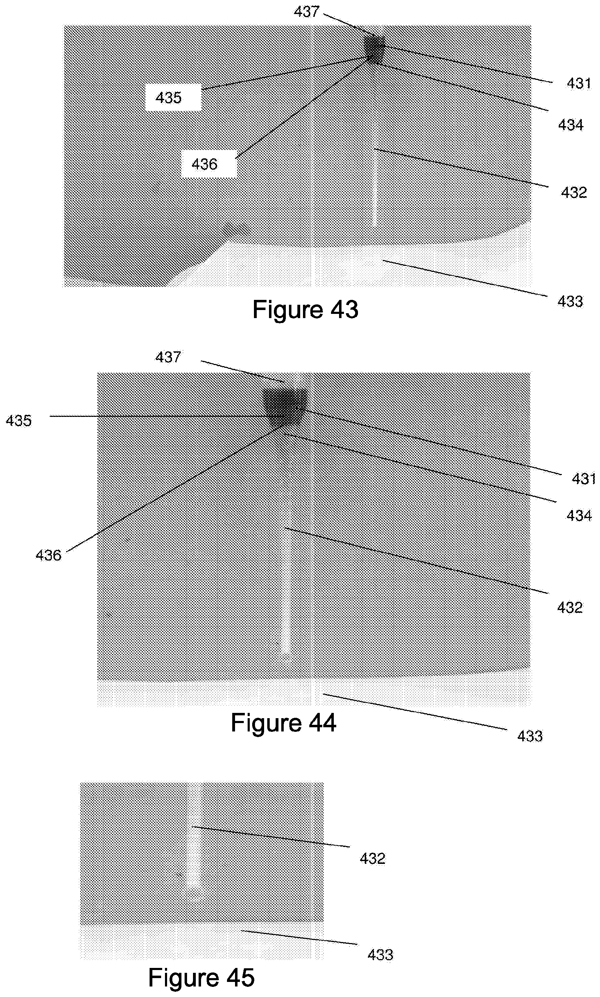

Figures 43, 44 and 45 show an experiment in which a dye/water solution 435 is passed over porous microspheres according to the invention 434. Figures 44 and 45 are magnified views of portions of Figure 43. A vertically-oriented glass micropipette 437 having an upper, wider portion 431 and a lower, thinner portion 432 is arranged above a blotting surface 433. A tapered portion 436 connects the upper, wider portion 431 to the lower, thinner portion 432. The porous microspheres according to the invention 434 are provided within the tapered portion 436. In the experiment, the dye/water solution 435 is passed through the micropipette 437.

In contrast with the experiments shown in Figures 41 and 42, the dye/water solution 435 is separated as it passes through the porous microspheres according to the invention 434. As can be seen in Figures 43, 44 and 45, the dye is retained in the upper, wider portion 431 of the micropipette 437 and only water exits the bottom of the micropipette 437 on to the blotting surface 435.

Accordingly, the porous microspheres according to the invention are capable of separating and retaining the dye from the water. This result suggests that with careful control over the chemistry and/or composition of the porous microspheres, e.g. porous glass or glass-ceramic microspheres, the internal surfaces of the porous microspheres could be adapted to filter out specific entities, e.g. heavy metals or other unwanted entities in solutions.

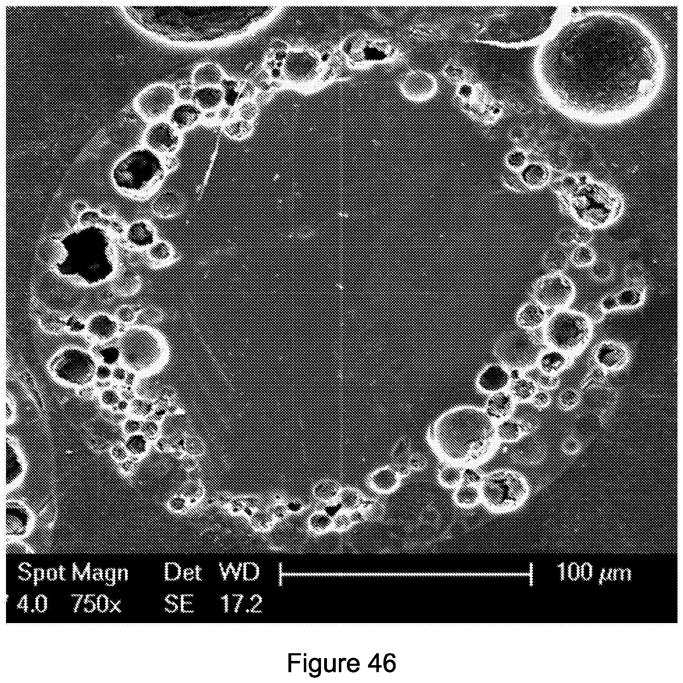

Figure 46 shows a polished cross-section of a calcium phosphate glass microsphere according to the invention. The calcium phosphate glass microsphere has a solid core and surface porosity.

## Claims

1. A method of manufacture of a powder comprising, or consisting essentially of, porous microspheres, the method comprising:
providing a feed powder;
mixing the feed powder with one or more blowing agents to provide a mixture comprising feed powder particles and particles of one or more blowing agents; and
applying at least one spheroidisation flame to the mixture, so as to produce the powder.

2. A method according to claim 1 wherein the microspheres produced comprise surface porosity and/or at least some interconnected porosity.

3. A method according to claim 1 or claim 2, wherein the feed powder comprises porous and/or non-porous particles, and/or wherein the feed powder comprises substantially spherical and/or non-spherical particles, and/or wherein the feed powder has an average particle size of at least 30 µm and up to 500 µm.

4. A method according to any preceding claim, wherein the blowing agent coats particles of the feed powder, and/or wherein the or each blowing agent has an average particle size of at least 5 µm and/or up to 500 µm, and/or wherein the or each blowing agent comprises a carbonate or a sulphate, e.g. calcium carbonate, strontium carbonate or sodium sulphate, and/or wherein the ratio by weight of the blowing agent(s) to the feed particles is from 5:1 to 1:10.

5. A method according to any one of the preceding claims, wherein the mixture is produced prior to applying the spheroidisation flame or at the same time as applying the spheroidisation flame, and/or wherein the spheroidisation flame temperature is from 1900 °C to 3400 °C.

6. A method according to any one of the preceding claims, the method comprising the step of soaking the feed powder in a solution containing the blowing agent(s) and/or coating the feed powder with the blowing agent(s), and/or a washing step to remove residual blowing agent(s) wherein the washing step optionally comprises washing the microspheres in acidic solution.

7. A powder manufactured by the method according to any one of claims 1 to 6, the powder comprising, or consisting essentially of, porous microspheres, wherein the microspheres comprise a glass, a glass-ceramic or a ceramic composition and wherein the microspheres have a total porosity of at least 40%.

8. A powder according to claim 7, or a method according to any one of claims 1 to 6, wherein any one or more of the following applies:
a) wherein the microspheres comprise a surface porosity and/or at least some interconnected porosity,
b) the microspheres have an average particle size of at least 30 µm and up to 500 µm, and/or
c) the microspheres comprise a resorbable composition or a non-resorbable composition.

9. A powder according to claim 7 or claim 8, wherein the microspheres comprise a phosphate-based glass such as a calcium phosphate-based glass, hydroxyapatite, α tri-calcium phosphate (α-TCP), β tri-calcium phosphate (β-TCP), a borosilicate glass, a borate glass or apatite wollastonite.

10. A powder according to any one of claims 7 to 9, wherein any one or more of the following applies:
a) the microspheres have a surface area per unit mass of at least 0.05 m²/g, and/or wherein the microspheres have a surface area per unit mass of up to or at least 0.08 m²/g, up to or at least 0.12 m²/g or up to or at least 0.14 m²/g, and/or
b) the microspheres have an average pore diameter of at least 10 µm and/or up to 100 µm, and/or
c) the microspheres have a total porosity of at least 50%, at least 60%, at least 70% or at least 80%.

11. A powder according to any one of claims 7 to 10, wherein the microspheres are coated and/or loaded with at least one active agent such as a pharmaceutically active agent, biological cells, e.g. stem cells, growth factors or proteins, and/or wherein the microspheres are coated and/or impregnated/doped with an anti-microbial agent such as at least one of silver, zinc and/or copper, and/or wherein the microspheres contain strontium, optionally in an amount of up to around 7 wt%.

12. A powder according to any one of claims 7 to 11, wherein the microspheres have only surface porosity or at least some interconnected porosity.

13. A powder according to any one of claims 7 to 12, wherein the powder comprises a mixture of a first powder and at least one further powder, wherein the first powder comprises microspheres having a first size distribution and the or each further powder comprises microspheres having a different size distribution.

14. A method of manufacture of a powder comprising, or consisting essentially of, microspheres, the method comprising:
manufacturing a first powder according to the method of any one of claims 1 to 6;
manufacturing at least one further powder according to the method of any one of claims 1 to 6, wherein the at least one further powder contains particles having a different size distribution and/or a different porosity from the first powder; and
mixing the first powder and the at least one further powder together.

15. A powder according to claim 13, wherein the microspheres of the first powder are smaller than the microspheres of a second powder, optionally wherein the microspheres of the first powder have an average particle size of up to 200 µm and/or the microspheres of the second powder have an average particle size of more than 200 µm.

## Patentansprüche

1. Verfahren zur Herstellung eines Pulvers, umfassend, oder im Wesentlichen bestehend aus, porösen Mikrokugeln, wobei das Verfahren Folgendes umfasst:
Bereitstellen eines Zufuhrpulvers;
Mischen des Zufuhrpulvers mit einem oder mehreren Treibmitteln, um eine Mischung bereitzustellen, die Partikel des Zufuhrpulvers und Partikel von einem oder mehreren Treibmitteln umfasst; und
Aufbringen mindestens einer Sphäroidisierungsflamme auf die Mischung, um das Pulver zu produzieren.

2. Verfahren nach Anspruch 1, wobei die produzierten Mikrokugeln Oberflächenporosität und/oder zumindest teilweise Porosität, die miteinander verbunden ist, umfassen.

3. Verfahren nach Anspruch 1 oder Anspruch 2, wobei das Zufuhrpulver poröse und/oder nicht-poröse Partikel umfasst und/oder wobei das Zufuhrpulver im Wesentlichen sphärische und/oder nicht-sphärische Partikel umfasst und/oder wobei das Zufuhrpulver eine durchschnittliche Partikelgröße von mindestens 30 µm und bis zu 500 µm aufweist.

4. Verfahren nach einem der vorhergehenden Ansprüche, wobei das Treibmittel die Partikel des Zufuhrpulvers beschichtet und/oder wobei das oder jedes Treibmittel eine durchschnittliche Partikelgröße von mindestens 5 µm und/oder bis zu 500 µm aufweist und/oder wobei das oder jedes Treibmittel ein Carbonat oder ein Sulfat, z. B. Calciumcarbonat, Strontiumcarbonat oder Natriumsulfat, umfasst und/oder wobei das Gewichtsverhältnis des/der Treibmittel(s) zu den Zufuhrpartikeln von 5:1 bis 1:10 reicht.

5. Verfahren nach einem der vorhergehenden Ansprüche, wobei die Mischung vor dem Aufbringen der Sphäroidisierungsflamme oder gleichzeitig mit dem Aufbringen der Sphäroidisierungsflamme produziert wird und/oder wobei die Temperatur der Sphäroidisierungsflamme von 1900 °C bis 3400 °C reicht.

6. Verfahren nach einem der vorhergehenden Ansprüche, wobei das Verfahren den Schritt Einweichen des Zufuhrpulvers in einer Lösung, die das/die Treibmittel enthält, und/oder Beschichten des Zufuhrpulvers mit dem/den Treibmittel(n) und/oder einen Waschschritt, um restliche(s) Treibmittel zu entfernen, umfasst, wobei der Waschschritt optional Waschen der Mikrokugeln in einer sauren Lösung umfasst.

7. Pulver, das mittels des Verfahrens nach einem der Ansprüche 1 bis 6 hergestellt ist, wobei das Pulver Folgendes umfasst oder im Wesentlichen aus Folgendem besteht: porösen Mikrokugeln, wobei die Mikrokugeln eine Glas-, eine GlasKeramik- oder eine Keramikzusammensetzung umfassen und wobei die Mikrokugeln eine Gesamtporosität von mindestens 40 % aufweisen.

8. Pulver nach Anspruch 7 oder Verfahren nach einem der Ansprüche 1 bis 6, wobei eines oder mehrere der Folgenden gelten:
a) wobei die Mikrokugeln Oberflächenporosität und/oder zumindest teilweise Porosität, die miteinander verbunden ist, umfassen,
b) die Mikrokugeln eine durchschnittliche Partikelgröße von mindestens 30 µm und bis zu 500 µm aufweisen und/oder
c) die Mikrokugeln eine resorbierbare Zusammensetzung oder eine nicht-resorbierbare Zusammensetzung umfassen.

9. Pulver nach Anspruch 7 oder Anspruch 8, wobei die Mikrokugeln ein phosphatbasiertes Glas wie beispielsweise ein calciumphosphatbasiertes Glas, Hydroxylapatit, α-Tricalciumphosphat (α-TCP), β-Tricalciumphosphat (β-TCP), ein Borosilikatglas, ein Boratglas oder Apatit-Wollastonit umfassen.

10. Pulver nach einem der Ansprüche 7 bis 9, wobei eines oder mehrere der Folgenden gelten:
a) wobei die Mikrokugeln einen Oberflächenbereich pro Masseneinheit von mindestens 0,05 m²/g aufweisen und/oder wobei die Mikrokugeln einen Oberflächenbereich pro Masseneinheit von bis zu oder mindestens 0,08 m²/g, bis zu oder mindestens 0,12 m²/g oder bis zu oder mindestens 0,14 m²/g aufweisen und/oder
b) die Mikrokugeln einen durchschnittlichen Porendurchmesser von mindestens 10 µm und/oder bis zu 100 µm aufweisen und/oder
c) die Mikrokugeln eine Gesamtporosität von mindestens 50 %, mindestens 60 %, mindestens 70 % oder mindestens 80 % aufweisen.

11. Pulver nach einem der Ansprüche 7 bis 10, wobei die Mikrokugeln mit mindestens einem Wirkstoff wie einem pharmazeutischen Wirkstoff, biologischen Zellen, z. B. Stammzellen, Wachstumsfaktoren oder Proteinen beschichtet und/oder beladen sind und/oder wobei die Mikrokugeln mit einem antimikrobiellen Mittel wie mindestens einem von Silber, Zink und/oder Kupfer beschichtet und/oder imprägniert/dotiert sind und/oder wobei die Mikrokugeln Strontium enthalten, optional in einer Menge von bis zu etwa 7 Gew.-%.

12. Pulver nach einem der Ansprüche 7 bis 11, wobei die Mikrokugeln nur Oberflächenporosität oder zumindest teilweise Porosität, die miteinander verbunden ist, aufweisen.

13. Pulver nach einem der Ansprüche 7 bis 12, wobei das Pulver eine Mischung aus einem ersten Pulver und mindestens einem weiteren Pulver umfasst, wobei das erste Pulver Mikrokugeln umfasst, die eine erste Größenverteilung aufweisen, und das oder jedes weitere Pulver Mikrokugeln umfasst, die eine unterschiedliche Größenverteilung aufweisen.

14. Verfahren zur Herstellung eines Pulvers, umfassend, oder im Wesentlichen bestehend aus, Mikrokugeln, wobei das Verfahren Folgendes umfasst:
Herstellen eines ersten Pulvers gemäß dem Verfahren nach einem der Ansprüche 1 bis 6;
Herstellen mindestens eines weiteren Pulvers gemäß dem Verfahren nach einem der Ansprüche 1 bis 6, wobei das mindestens eine weitere Pulver Partikel enthält, die eine unterschiedliche Größenverteilung und/oder eine unterschiedliche Porosität als das erste Pulver aufweisen; und
Zusammenmischen des ersten Pulvers und des mindestens einen weiteren Pulvers.

15. Pulver nach Anspruch 13, wobei die Mikrokugeln des ersten Pulvers kleiner sind als die Mikrokugeln eines zweiten Pulvers, optional wobei die Mikrokugeln des ersten Pulvers eine durchschnittliche Partikelgröße von bis zu 200 µm aufweisen und/oder die Mikrokugeln des zweiten Pulvers eine durchschnittliche Partikelgröße von mehr als 200 µm aufweisen.

## Revendications

1. Procédé de fabrication d'une poudre comprenant, ou constituée essentiellement de, microsphères poreuses, le procédé comprenant :
la fourniture d'une poudre de charge ;
le mélange de la poudre de charge avec un ou plusieurs agents gonflants pour fournir un mélange comprenant des particules de poudre de charge et des particules d'un ou plusieurs agents gonflants ; et
l'application d'au moins une flamme de sphéroïdisation au mélange, de manière à produire la poudre.

2. Procédé selon la revendication 1, dans lequel les microsphères produites comprennent une porosité de surface et/ou au moins une certaine porosité interconnectée.

3. Procédé selon la revendication 1 ou la revendication 2, dans lequel la poudre de charge comprend des particules poreuses et/ou non poreuses, et/ou dans lequel la poudre de charge comprend des particules sensiblement sphériques et/ou non sphériques, et/ou dans lequel la poudre de charge a une taille moyenne de particule d'au moins 30 µm et allant jusqu'à 500 µm.

4. Procédé selon l'une quelconque des revendications précédentes, dans lequel l'agent gonflant revêt des particules de la poudre de charge, et/ou dans lequel le ou chaque agent gonflant a une taille moyenne de particule d'au moins 5 µm et/ou allant jusqu'à 500 µm, et/ou dans lequel le ou chaque agent gonflant comprend un carbonate ou un sulfate, par exemple du carbonate de calcium, du carbonate de strontium ou du sulfate de sodium, et/ou dans lequel le rapport en poids du ou des agents gonflants aux particules de charge est de 5:1 à 1:10.

5. Procédé selon l'une quelconque des revendications précédentes, dans lequel le mélange est produit avant l'application de la flamme de sphéroïdisation ou en même temps que l'application de la flamme de sphéroïdisation, et/ou dans lequel la température de la flamme de sphéroïdisation est de 1900 °C à 3400 °C.

6. Procédé selon l'une quelconque des revendications précédentes, le procédé comprenant l'étape consistant à faire tremper la poudre de charge dans une solution contenant les un ou plusieurs agents gonflants et/ou l'étape consistant à revêtir la poudre de charge avec les un ou plusieurs agents gonflants, et/ou une étape de lavage pour éliminer les un ou plusieurs agents gonflants résiduels, dans lequel l'étape de lavage comprend éventuellement le lavage des microsphères dans une solution acide.

7. Poudre fabriquée par le procédé selon l'une quelconque des revendications 1 à 6, la poudre comprenant, ou constituée essentiellement de, microsphères poreuses, dans laquelle les microsphères comprennent une composition de verre, de vitrocéramique ou de céramique et dans laquelle les microsphères ont une porosité totale d'au moins 40 %.

8. Poudre selon la revendication 7, ou procédé selon l'une quelconque des revendications 1 à 6, dans laquelle ou dans lequel l'un quelconque ou plusieurs des points suivants s'appliquent :
a) dans laquelle les microsphères comprennent une porosité de surface et/ou au moins une certaine porosité interconnectée,
b) les microsphères ont une taille moyenne de particule d'au moins 30 µm et allant jusqu'à 500 µm, et/ou
c) les microsphères comprennent une composition résorbable ou une composition non résorbable.

9. Poudre selon la revendication 7 ou la revendication 8, dans laquelle les microsphères comprennent un verre à base de phosphate tel qu'un verre à base de phosphate de calcium, de l'hydroxyapatite, du phosphate tricalcique α (α-TCP), du phosphate tricalcique β (β-TCP), un verre de borosilicate, un verre de borate ou de l'apatite wollastonite.

10. Poudre selon l'une quelconque des revendications 7 à 9, dans laquelle l'un quelconque ou plusieurs des points suivants s'appliquent :
a) les microsphères ont une surface spécifique par unité de masse d'au moins 0,05 m²/g, et/ou dans laquelle les microsphères ont une surface spécifique par unité de masse allant jusqu'à ou d'au moins 0,08 m²/g, allant jusqu'à ou d'au moins 0,12 m²/g ou allant jusqu'à ou d'au moins 0,14 m²/g, et/ou
b) les microsphères ont un diamètre moyen de pore d'au moins 10 µm et/ou allant jusqu'à 100 µm, et/ou
c) les microsphères ont une porosité totale d'au moins 50 %, d'au moins 60 %, d'au moins 70 % ou d'au moins 80 %.

11. Poudre selon l'une quelconque des revendications 7 à 10, dans laquelle les microsphères sont revêtues et/ou chargées avec au moins un agent actif tel qu'un agent biologiquement actif, des cellules biologiques, par exemple des cellules souches, des facteurs de croissance ou des protéines, et/ou dans laquelle les microsphères sont revêtues et/ou imprégnées/dopées avec un agent antimicrobien tel qu'au moins l'un parmi l'argent, le zinc et/ou le cuivre, et/ou dans laquelle les microsphères contiennent du strontium, éventuellement en une quantité allant jusqu'à environ 7 % en poids.

12. Poudre selon l'une quelconque des revendications 7 à 11, dans laquelle les microsphères ont uniquement une porosité de surface ou au moins une certaine porosité interconnectée.

13. Poudre selon l'une quelconque des revendications 7 à 12, dans laquelle la poudre comprend un mélange d'une première poudre et d'au moins une poudre supplémentaire, dans laquelle la première poudre comprend des microsphères ayant une première distribution de taille et la ou chaque poudre supplémentaire comprend des microsphères ayant une distribution de taille différente.

14. Procédé de fabrication d'une poudre comprenant, ou constituée essentiellement de, microsphères, le procédé comprenant :
la fabrication d'une première poudre selon le procédé de l'une quelconque des revendications 1 à 6 ;
la fabrication d'au moins une poudre supplémentaire selon le procédé de l'une quelconque des revendications 1 à 6, dans lequel l'au moins une poudre supplémentaire contient des particules ayant une distribution de taille différente et/ou une porosité différente de la première poudre ; et
le mélange de la première poudre et de l'au moins une poudre supplémentaire ensemble.

15. Poudre selon la revendication 13, dans laquelle les microsphères de la première poudre sont plus petites que les microsphères d'une deuxième poudre, éventuellement dans laquelle les microsphères de la première poudre ont une taille moyenne de particule allant jusqu'à 200 µm et/ou les microsphères de la deuxième poudre ont une taille moyenne de particule de plus de 200 µm.
